# EUROPEAN PATENT SPECIFICATION

(11) **EP 2 355 838 B1**
(45) Date of publication and mention of the grant of the patent: **03.09.2014**
(21) Application number: 09774707.5
(22) Date of filing: 07.11.2009
(51) Int. Cl.: A61K 38/17, A61P 3/08, A61P 3/04, A61P 3/10, A61P 5/50, A61P 21/00, A61K 31/135, A61K 31/195, A61K 45/06

(54) **GLYCOPROTEINS HAVING LIPID MOBILIZING PROPERTIES AND THERAPEUTIC USES THEREOF**
GLYKOPROTEINE MIT LIPIDMOBILISIERENDEN EIGENSCHAFTEN UND THERAPEUTISCHE VERWENDUNGEN DAVON
GLYCOPROTÉINES DOTÉES DE PROPRIÉTÉS MOBILISATRICES DES LIPIDES ET LEURS UTILISATIONS THÉRAPEUTIQUES

(30) Priority: 07.11.2008 US 112623 P; 19.10.2009 US 253023 P
(43) Date of publication of application: 17.08.2011
(73) Proprietor: Aston University, Birmingham B4 7ET (GB)
(72) Inventor: TISDALE, Michael, J., Claverdon Warwickshire CV35 8LW (GB); RUSSELL, Steven, Wedensbury West Midlands WS10 7RR (GB)
(74) Representative: Lau, Sarah Jane
(86) International application number: PCT/IB2009/007373
(87) International publication number: WO 2010/052563

(56) References cited:
- WO-A1-02/18436
- WO-A1-94/24090
- WO-A1-94/29290
- WO-A2-03/050281
- US-A- 5 480 908
- US-A1- 2003 040 538
- US-B1- 6 657 063
- US-B1- 6 890 899
- RUSSELL S T ET AL: "Induction of lipolysis in vitro and loss of body fat in vivo by zinc-alpha2-glycoprotein" BIOCHIMICA AND BIOPHYSICA ACTA. MOLECULAR AND CELL BIOLOGY OFLIPIDS, ELSEVIER, AMSTERDAM, NL, vol. 1636, no. 1, 27 February 2004 (2004-02-27), pages 59-68, XP004492622 ISSN: 1388-1981
- DATABASE WPI Week 199710 Thomson Scientific, London, GB; AN 1997-102219 XP002590085 & ES 2 094 687 A1 (LAB SALVA SA) 16 January 1997 (1997-01-16)
- RUSSELL S T ET AL: "The role of glucocorticoids in the induction of zinc-alpha2-glycoprotein expression in adipose tissue in cancer cachexia" BRITISH JOURNAL OF CANCER, vol. 92, no. 5, 14 March 2005 (2005-03-14), pages 876-881, XP002590047 ISSN: 0007-0920
- SANDERS PAUL M ET AL: "Effect of zinc-alpha2-glycoprotein (ZAG) on expression of uncoupling proteins in skeletal muscle and adipose tissue." CANCER LETTERS LNKD- PUBMED:15246563, vol. 212, no. 1, 20 August 2004 (2004-08-20), pages 71-81, XP002590048 ISSN: 0304-3835

## Description

### BACKGROUND OF THE INVENTION

### FIELD OF THE INVENTION

The present invention relates generally to medicine and the treatment of hyperglycemia and obesity, and more particularly, to compositions and methods for ameliorating symptoms associated with hyperglycemia.

### BACKGROUND INFORMATION

Diabetes is characterized by impaired glucose metabolism manifesting itself among other things by an elevated blood glucose level in the diabetic patient. Underlying defects lead to a classification of diabetes into two major groups. Type 1 diabetes, or insulin dependent diabetes mellitus (IDDM), arises when patients lack insulin-producing beta-cells in their pancreatic glands. Type 2 diabetes, or non-insulin dependent diabetes mellitus (NIDDM), *occurs* in patients with impaired beta-cell function and alterations in insulin action.

Both type 1 and type 2 diabetes are associated with loss of skeletal muscle mass, which has been attributed to an increased break-down of myofibrillar proteins, through an increased activity of the ubiquitin-proteasome pathway. Although this is particularly prevalent in type 1 diabetes, it is only partly explained by insulin deprivation, and experimental studies in db/db mice show that insulin resistance causes muscle wasting.

Loss of skeletal muscle occurs in models of both severely hypoinsulinemic (streptozotocin-induced) and in severely insulin resistant (ob/ob) diabetes in mice. Muscle protein loss in diabetes is associated with both a depression of protein synthesis, and an increase in protein degradation in skeletal muscle. The increase in protein degradation is associated with increased activity of both caspase-3 and the proteasome.

Obesity is associated with insulin resistance and type 2 diabetes. An impaired catecholamine-induced lipolysis and reduced hormone sensitive lipase (HSL) expression is observed in adipocytes in obesity, which has been suggested to contribute to the development, or maintenance, of increased adipose tissue stores. In contrast adipocytes from cachexic subjects show a two- to three-fold increase in the lipolytic response with increased expression of HSL.

Zinc-α₂-glycoprotein (ZAG) has been identified as a lipid mobilizing factor (LMF) with the potential to induce fat loss in cancer cacehxia. ZAG was shown to induce lipolysis in white adipocytes by interaction with a β3-adrenergic receptor, while *in vivo* it increased expression of uncoupling protein-1 (UCP-1) in brown adipose tissue (BAT), and induced loss of body fat. In addition to some tumors ZAG is also produced by white adipose tissue (WAT) and BAT and its expression is upregulated in cachexia. In contrast ZAG expression in adipose tissue of obese humans was only 30% of that found in non-obese subjects. This suggests that loss of ZAG expression in WAT could account for some of the features of obesity. Certainly inactivation of both ZAG alleles in mice led to an increase in body weight which was more pronounced when the animals were fed a high fat diet. The lipolytic response to various agents was significantly decreased in adipocytes from ZAG deficient animals.

To date all studies on the lipid mobilizing effect of ZAG have been carried out in mice, using human ZAG, although the identities in amino acid sequence of ZAG between mouse and human is only 58.6%. Since the sequence homology between rat and mouse is 88.5%, this suggests that if human ZAG binds to the mouse receptor it should also be effective in the rat. The current study investigates the anti-obesity effect of human ZAG in mature male Wistar rats.

US6890899 discloses that a polypeptide called lipid mobilising factor reduced serum glucose levels in an *in vivo* animal model.

WO03/050281 discloses that Genset ZAG interacting protein may be used to lower blood glucose levels.

### SUMMARY OF THE INVENTION

The present invention is based on the finding that Zinc-α₂-glycoprotein (ZAG) has an effect on body weight and insulin responsiveness in adult obese hyperglycemic (ob/ob) mice and mature Wistar rats, as models of type 2 diabetes. Such a finding is useful in methods for ameliorating the symptoms associated with hyperglycemia.

Thus the present invention relates to a polypeptide having the sequence as shown in SEQ ID NO: 1 for use in ameliorating symptoms of hyperglycemia by administering to the subject a therapeutically effective dosage for a period of 21 days or longer, wherein there is a continuous amelioration of symptoms of hyperglycemia over the course of treatment.

Accordingly, the present disclosure provides a method of ameliorating symptoms of hyperglycemia in a subject. The method includes administering to the subject in need of such treatment a therapeutically effective dosage of a polypeptide having the sequence as shown in SEQ ID NO: 1 for a period of about 21 days or longer, resulting in an amelioration of symptoms associated with hyperglycemia following treatment. The polypeptide may be administered twice daily. The polypeptide may be administered intravenously, subcutaneously, intraperitoneally, or orally. The polypeptide may be administered in combination with one or more agents selected from the group consisting of a β3-adrenergic receptor (β3-AR) antagonist and a β3 agonist. In one option, the β3-AR antagonist is SR59230A. In another option, β3 agonist is AMNI-BRL37344 (BRL37344). In another option, the polypeptide is glycosylated.

Amelioration of the symptoms associated with hyperglycemia includes, but is not limited to, a decrease in serum levels of glucose, a decrease in serum levels of triglycerides, a decrease in serum levels of insulin, and a decrease in serum levels of non-esterified fatty acids, as compared to serum levels prior to treatment. In one embodiment, the amelioration of symptoms includes an increase in body temperature of about 0.4° to 1°C within 1 day of initiating treatment, as compared to body temperature prior to treatment. In another embodiment, the amelioration of symptoms includes a decrease in plasma insulin levels within 3 days of initiating treatment, as compared to plasma insulin levels prior to treatment. Plasma insulin and/or glucose and triglyceride levels may be decreased as much as 36% as compared to plasma insulin and/or glucose and triglyceride levels prior to treatment. In another embodiment, the amelioration of symptoms comprises normalization of blood glucose levels and insulin secretion in response to intravenous glucose (2g/kg) within 3 days of initiating treatment. In another embodiment, the amelioration of symptoms comprises an increase in pancreatic insulin levels, as compared to pancreatic insulin levels prior to treatment. In yet another embodiment, the amelioration of symptoms comprises increased expression of uncoupling protein-1 (UCP1) and uncoupling protein-3 (UCP3) in brown adipose tissue, as compared to expression of UCP1 and UCP3 prior to treatment. In yet another embodiment, the amelioration of symptoms comprises increased expression of UCP3 in skeletal muscle, as compared to expression of UCP3 prior to treatment. In another embodiment, the polypeptide is glycosylated.

In another aspect, the present disclosure provides a method of decreasing plasma insulin levels in a subject. The method includes administering to the subject a therapeutically effective dosage of a polypeptide having the sequence as shown in SEQ ID NO: 1. The decrease in plasma insulin levels may occur within 3 days of administering the polypeptide. In another option, the polypeptide is administered in combination with one or more agents selected from the group consisting of a β3-adrenergic receptor (β3-AR) antagonist and a β3 agonist. In one option, the β3-AR antagonist is SR59230A. In another option β3 agonist is AMNI-BRL37344 (BRL37344). In another option the polypeptide is glycosylated.

In another aspect, the present disclosure provides a method of increasing skeletal muscle mass in a subject. The method includes administering to the subject a polypeptide having the sequence as shown in SEQ ID NO: 1. In one embodiment, the skeletal muscle is a gastrocnemius muscle or a soleus muscle. In another option the polypeptide is administered in combination with one or more agents selected from the group consisting of a β3-adrenergic receptor (β3-AR) antagonist and a β3 agonist. In one option the β3-AR antagonist is SR59230A. In another option β3 agonist is AMNI-BRL37344 (BRL37344). In another option the polypeptide is glycosylated.

In another aspect, the present disclosure provides a method of treating a subject to bring about a weight reduction or reduction in obesity. The method includes administering to the subject in need of such treatment a therapeutically effective dosage of a polypeptide having the sequence as shown in SEQ ID NO: 1 In combination with one or more agents selected from the group consisting of a β3-adrenergic receptor (β3-AR) antagonist and a β3 agonist. In one form the β3-AR antagonist is SR59230A. In another form, β3 agonist is AMNI-BRL37344 (BRL37344). In another form, the polypeptide is glycosylated.

In another aspect, the present disclosure provides a method of treating a subject to bring about a weight reduction or reduction in obesity. The method includes administering to the subject in need of such treatment a therapeutically effective dosage of a polypeptide having the sequence as shown in SEQ ID NO: 1 in combination with one or more agents selected from the group consisting of a β3-adrenergic receptor (β3-AR) antagonist and a β3 agonist. In one form the β3-AR antagonist is SR59230A. In another form β3 agonist is AMNI-BRL37344 (BRL37344). In another form the polypeptide is glycosylated.

In another aspect, the present invention disclosure provides a pharmaceutical composition comprising a polypeptide having the sequence as shown in SEQ ID NO: 1 and an agent selected from the group consisting of a β3-adrenergic receptor (β3-AR) antagonist and a β3 agonist. In one form the β3-AR antagonist is SR59230A. In another form, β3 agonist is AMNI-BRL37344 (BRL37344). In another form, the polypeptide is glycosylated.

### BRIEF DESCRIPTION OF THE DRAWINGS

Figure 1A is a pictorial diagram showing characterization of ZAG and its effect on lipolysis and body weight of ob/ob mice. Coomassie staining after 12% SDS-PAGE showing total proteins in 293 cell media and ZAG purified as described.
Figure 1B is a pictorial diagram showing the results of a Western blot showing expression of ZAG in culture medium and purified ZAG.
Figure 1C is a graphical diagram showing ZAG mRNA levels in adipose tissue and liver tissue in MAC16 mice undergoing weight loss. P<0.01.
Figure 1D is a graphical diagram showing the results of lipolysis in epididymal adipocytes from non-obese (■) and ob/ob mice (□) in response to isoprenaline (Iso) and ZAG. Differences from non-obese mice are shown as *p<0.05, **p<0.01 and ***p<0.001.
Figure 1E is a graphical diagram showing the results of lipolysis in adipocytes from epididymal (ep), subcutaneous (s.c.) and visceral (vis) deposits from obese (ob/ob) and non-obese (non ob) mice with either no treatment (■), isoprenaline (10µM) (□) or ZAG (0.46µM) (). Differences from epididymal adipocytes are shown as ** p<0.01.
Figure 1F is a graphical diagram showing the effect of ZAG (■) on body weight of ob/ob mice in comparison with PBS (◆) as described in the methods. Differences in weight form time zero and PBS controls are shown as ***p<0.001.
Figure 1G is a graphical diagram showing the effect of ZAG (□) on body temperature of the mice shown in e in comparison with PBS controls (■). Differences from control are shown as ***p<0.001.
Figure 2A is a graphical diagram showing glucose tolerance, glucose uptake into adipocytes and gastrocnemius muscle of ob/ob mice treated with ZAG. Plasma glucose levels in ob/ob mice in the fed state either treated with ZAG (■) or PBS (◆) for 3 days after i.v. administration of glucose (2g/kg). p<0.001 from PBS. Blood samples were removed from the tail vein at intervals after glucose administration and used for the measurement of glucose and insulin.
Figure 2B is a graphical diagram showing plasma insulin levels in ob/ob mice treated with ZAG after oral administration of glucose (1g/kg). p<0.001 from PBS.
Figure 2C is a graphical diagram showing glucose uptake into epididymal (ep), visceral (vis) and subcutaneous (s.c.) adipocytes of ob/ob mice treated with ZAG for 5 days in the presence of 0 (■), 1(□) or 10nM insulin (). Differences in the presence of ZAG are indicated as ***p<0.001.
Figure 2D is a graphical diagram showing uptake of 2-deoxy-D-glucose into gastrocnemius muscle of ob/ob mice treated with either ZAG or PBS for 5 days in the absence or presence of insulin (100nM). Differences in the presence of insulin are shown as *p<0.05 or **p<0.01, while differences in the presence of ZAG are shown as ***p<0.001.
Figure 2E is a pictorial diagram showing the effect of ZAG on protein synthesis and degradation in skeletal muscle of ob/ob mice. After treatment of ob/ob mice for 5 days skeletal muscle was removed and Western blotted for expression of GLUT4.
Figure 3A is a graphical diagram showing the effect of ZAG on protein synthesis and degradation in skeletal muscle of ob/ob mice. After treatment of ob/ob mice for 5 days skeletal muscle was removed and used for the measurement of protein synthesis. Differences from PBS controls, or non-obese animals are shown as ***p<0.001.
Figure 3B is a graphical diagram showing the effect of ZAG on protein synthesis and degradation in skeletal muscle of ob/ob mice. After treatment of ob/ob mice for 5 days skeletal muscle was removed and used for the measurement of protein degradation. Differences from PBS controls, or non-obese animals are shown as ***p<0.001.
Figure 3C is a graphical diagram showing the effect of ZAG on protein synthesis and degradation in skeletal muscle of ob/ob mice. After treatment of ob/ob mice for 5 days skeletal muscle was removed and used for the measurement of chymotrypsin-like enzyme activity. Differences from PBS controls, or non-obese animals are shown as ***p<0.001.
Figure 3D is a pictorial diagram showing the effect of ZAG on protein synthesis and degradation in skeletal muscle of ob/ob mice. After treatment of ob/ob mice for 5 days skeletal muscle was removed and Western blotted for expression of 20S-proteasome α-subunits.
Figure 3E is a pictorial diagram showing the effect of ZAG on protein synthesis and degradation in skeletal muscle of ob/ob mice. After treatment of ob/ob mice for 5 days skeletal muscle was removed and Western blotted for expression of p42.
Figure 3F is a pictorial diagram showing the effect of ZAG on protein synthesis and degradation in skeletal muscle of ob/ob mice. After treatment of ob/ob mice for 5 days skeletal muscle was removed and Western blotted for expression of myosin.
Figure 3G is a pictorial diagram showing the effect of ZAG on protein synthesis and degradation in skeletal muscle of ob/ob mice. After treatment of ob/ob mice for 5 days skeletal muscle was removed and Western blotted for expression of actin.
Figure 4A is a pictorial diagram showing the effect of ZAG on catabolic signaling pathways in skeletal muscle by Western blotting of phospho PKR in gastrocnemius muscle of ob/ob mice after treatment with either PBS or ZAG for 5 days. The total forms of the proteins serve as loading controls. Differences from PBS controls are shown as ***p<0.001 while differences from non-obese mice are shown as # p<0.001.
Figure 4B is a pictorial diagram showing the effect of ZAG on catabolic signaling pathways in skeletal muscle by Western blotting of phospho eIF2a in gastrocnemius muscle of ob/ob mice after treatment with either PBS or ZAG for 5 days. The total forms of the proteins serve as loading controls. Differences from PBS controls are shown as ***p<0.001 while differences from non-obese mice are shown as # p<0.001.
Figure 4C is a pictorial diagram showing the effect of ZAG on catabolic signaling pathways in skeletal muscle by Western blotting of phospho PLA₂ in gastrocnemius muscle of ob/ob mice after treatment with either PBS or ZAG for 5 days. The total forms of the proteins serve as loading controls. Differences from PBS controls are shown as ***p<0.001 while differences from non-obese mice are shown as # p<0.001.
Figure 4D is a pictorial diagram showing the effect of ZAG on catabolic signaling pathways in skeletal muscle by Western blotting of phospho p38MAPK in gastrocnemius muscle of ob/ob mice after treatment with either PBS or ZAG for 5 days. The total forms of the proteins serve as loading controls. Differences from PBS controls are shown as *** p<0.001 while differences from non-obese mice are shown as # p<0.001.
Figure 4E is a graphical diagram showing the effect of ZAG on catabolic signaling pathways in skeletal muscle by activity of caspase-3 (■) and caspase-8 (□)in gastrocnemius muscle of ob/ob mice after treatment with either PBS or ZAG for 5 days.
Figure 5A is a pictorial diagram showing expression of HSL and ATGL in response to ZAG. Western blots show expression of phospho HSL in adipocytes of non-obese mice 3h after no treatment (Con), or treatment with isoprenaline (10µM) or ZAG (0.46µM) alone, or in the presence of PD98059 (25µM) after 5 days treatment with ZAG.
Figure 5B is a pictorial diagram showing expression of HSL by immunoblotting in epididymal (ep) adipocytes after 5 days treatment with ZAG.
Figure 5C is a pictorial diagram showing expression of HSL by immunoblotting in subcutaneous (sc) adipocytes after 5 days treatment with ZAG.
Figure 5D is a pictorial diagram showing expression of HSL by immunoblotting in visceral (vis) adipocytes after 5 days treatment with ZAG.
Figure 5E is a pictorial diagram showing expression of ATGL in epididymal adipocytes after 5 days treatment with ZAG.
Figure 5F is a pictorial diagram showing expression of ATGL in subcutaneous adipocytes after 5 days treatment with ZAG.
Figure 5G is a pictorial diagram showing expression of ATGL in visceral adipocytes after 5 days treatment with ZAG.
Figure 5H is a pictorial diagram showing expression of ERK in epididymal adipocytes after 5 days treatment with ZAG.
Figure 5I is a pictorial diagram showing expression of ERK in subcutaneous adipocytes after 5 days treatment with ZAG.
Figure 5J is a pictorial diagram showing expression of ERK in visceral adipocytes after 5 days treatment with ZAG.
Figure 5K is a graphical diagram showing the response of adipocytes from epididymal (ep), subcutaneous (sc) and visceral (vis) deposits from ob/ob mice treated with either PBS or ZAG for 5 days to the lipolytic effect of BRL37344. Differences from PBS controls are indicated as ***p<0.01, while differences in the presence of PD98059 is shown as # p<0.001.
Figure 6A is a pictorial diagram showing the Effect of treatment of ob/ob mice for 5 days with ZAG on the expression of ZAG and HSL in WAT, UCP1 and UCP3 in BAT and UCP3 in gastrocnemius muscle. Western blot showing expression of ZAG in ep, sc, and vis adipocytes. Day 0 represents the day the adipocytes were removed from the mice.
Figure 6B is a pictorial diagram showing expression of ZAG in epididymal adipocytes that were suspended in RMPI medium as described in methods. The samples were then taken out at daily intervals and Western blotted for ZAG expression. Day 0 represents the day the adipocytes were removed from the mice.
Figure 6C is a pictorial diagram showing expression of HSL in epididymal adipocytes that were suspended in RMPI medium as described in methods. The samples were then taken out at daily intervals and Western blotted for HSL expression. Day 0 represents the day the adipocytes were removed from the mice.
Figure 6D is a pictorial diagram showing expression of UCP1 in BAT removed from mice. Differences from PBS treated mice are shown as ***p<0.001.
Figure 6E is a pictorial diagram showing expression of UCP3 in BAT removed from mice. Differences from PBS treated mice are shown as ***p<0.001.
Figure 6F is a pictorial diagram showing expression of UCP3 in gastrocnemius muscle removed from mice. Differences from PBS treated mice are shown as ***p<0.001.
Figure 7A is a graphical diagram showing weight loss of the ob/ob mice during the 21 day study. ZAG was injected at days 1, 4, 5, 8, 13, 16, 18, and 19; PBS was injected at the same time points.
Figure 7B is a graphical diagram showing weight change (g) of the ob/ob mice (weight 80-90g) during treatment with ZAG.
Figure 7C is a graphical diagram showing increased body temperature of the ob/ob mice during the 21 day study. ZAG was injected at days 1, 4, 5, 8, 13, 16, 18, and 19; PBS was injected at the same time points.
Figure 8A is a graphical diagram showing a progressive decrease in urinary glucose excretion during the first 5 days of treatment.
Figure 8B is a graphical diagram showing a progressive decrease in urinary glucose excretion during the 21 day study.
Figure 9 is a graphical diagram showing glycerol release stimulated by isoprenaline (iso) isolated adipocytes which have been in culture up to 5 days from ob mice treated with and without ZAG.
Figure 10 is a pictorial diagram showing the complete amino acid sequence (SEQ ID NO: 1) of the human plasma Zn-α₂-glycoprotein, as published by T. Araki et al. (1988) "Complete amino acid sequence of human plasma Zn-α₂-glycoprotein and its homology to histocompatibility antigens."
Figure 11 is a graphical diagram showing lipolytic activity of human ZAG in isolated rat epididymal adipocytes, compared with isoprenaline (10µM) in the absence or presence of SR59230A (10µM) or anti-ZAG antibody (1:1000) (IgG). Each value is an average of 5 separate studies. Differences from control are shown as b, p<0.01 or c, p<0.001, while differences from ZAG alone are indicated as e, p<0.01 or f, p<0.001.
Figure 12A is a graphical diagram showing the effect of daily i.v. administration of either ZAG (50µg/100g b.w.) in 100µl PBS (■) or PBS alone (◆) on body weight of male Wistar rats over a 10 day period. The protocol for the experiment is given in the methods section.
Figure 12B is a graphical diagram showing the body temperature of male Wistar rats administered either ZAG (■) or PBS (◆) as described in Figure 12A.
Figure 12C is a graphical diagram showing the uptake of 2-deoxy-D-glucose into epididymal adipocytes of male Wistar rats after 10 days treatment with either ZAG (open box) or PBS (closed box) for 10 days, as shown in Figure 12A, in the absence or presence of insulin (60µU/ml).
Figure 12D is a graphical diagram showing glucose uptake into gastrocnemius muscle and BAT of male Wistar rats after 10 days treatment with either ZAG or PBS, in the absence or presence of insulin (60µU/ml). Differences between ZAG and PBS treated animals are shown as a, p<0.05, b, p<0.01 or c, p<0.001, while differences in the presence of insulin are shown as or f, p<0.001.
Figure 12E is a graphical diagraph showing tissue Rg in ob/ob mice administered ZAG. c, p<0.001 from PBS.
Figures 13A-13C are pictorial diagrams of Western blots showing expression of GLUT4 in BAT (Figure 13A) and WAT (Figure 13B) and gastrocnemius muscle (Figure 13C) of male Wistar rats treated with either PBS or ZAG for 10 days as shown in Figure 12. Differences between ZAG and PBS treated animals are shown as c, p<0.001.
Figures 14A and 14B are pictorial diagrams of Western blots showing expression of UCP1 and UCP3 in BAT (Figure 14A) and WAT (Figure 14B) of male Wistar rats treated with either PBS or ZAG for 10 days as shown in Figure 12. Differences between ZAG and PBS treated animals are shown as c, p<0.001.
Figures 15A and 15B are pictorial diagrams of Western blots showing expression of ATGL (Figure 15A) and HSL (Figure 15B) in epididymal adipose tissue of male Wistar rats treated with either PBS or ZAG for 10 days as shown in Figure 12. Differences between ZAG and PBS treated animals are shown as c, p<0.001.
Figures 16A-16C are pictorial diagrams of Western blots showing expression of ZAG in gastrocnemius muscle (Figure 16A), WAT (Figure 16B) and BAT (Figure 16C). Tissues were excised from male Wistar rats treated with either PBS or ZAG for 10 days as shown in Figure 12. Differences between ZAG and PBS treated animals are shown as c, p<0.001.
Figures 17A and 17B are pictorial diagrams of Western blots showing expression of phosphorylated and total forms of PKR (Figure 17A) and eIF2α (Figure 17B) in gastrocnemius muscle of male Wistar rats treated with either PBS or ZAG for 10 days as shown in Figure 12. The densitometric analysis is the ratio of the phosphor to total forms, expressed as a percentage of the value for rats treated with PBS.
Figures 18A and 18B is a graphical diagram showing phenylalanine release (Figure 18A) and protein synthesis (Figure 18B) in C2C12 myotubes treated with and without ZAG for 4h in the presence of various concentrations of glucose. Statistically significant c, P<0.001 from control; f, P<0.001 from glucose alone.
Figure 19 is a graphical diagram showing pheylalanine release in C2C12 myotubes treated with and without ZAG in the presence of various concentrations of glucose and with and without SR59230A. Statistically significant b, P<0.01 and c, P<0.001 from control; e, P<0.05 and f, P<0.001 from glucose alone.
Figure 20 is a graphical diagram showing protein synthesis in C2C12 myotubes treated with and without ZAG in the presence of various concentrations of glucose and with and without SR59230A. Statistically significant b, P<0.01 and c, P<0.001 from control; e, P<0.05 and f, P<0.001 from glucose alone; I, P<0.001 from glucose+SR.
Figure 21 is a graphical diagram showing ROS activity in C2C12 myotubes treated with various concentrations of glucose with and without ZAG. Statistically significant c, P<0.001 from control f, P<0.001 from glucose alone.
Figure 22A is a pictorial diagram of a Western blot showing PKR in C2C12 myotubes treated with glucose with and without ZAG. Statistically significant c, P<0.001 from control f, P<0.001 from glucose alone.
Figure 22B is a pictorial diagram of a Western blot showing eIF2α in C2C12 myotubes treated with glucose with and without ZAG. Statistically significant c, P<0.001 from control f, P<0.001 from glucose alone.
Figures 23A and 23B are graphical diagrams showing the results of an insulin tolerance test in ob/ob mice treated with and without ZAG. Statistically significant b, P<0.05 and c, P<0.001 from with ZAG.
Figure 24 is a graphical diagram showing the oxidation of D-[U-¹⁴C glucose] to ¹⁴CO₂ in ob/ob mice.
Figure 25 is a graphical diagram showing production of ¹⁴CO₂ from [¹⁴C carboxy] triolein in ob/ob mice.

### DETAILED DESCRIPTION OF THE INVENTION

The present invention is based on the observation that recombinant human Zinc-α₂-glycoprotein (ZAG) produces a decrease in body weight and increase in insulin responsiveness in ob/ob mice and in Wistar rats with no effect on food intake. As such, the disclosure provides methods for ameliorating the symptoms of hyperglycemia in a subject, decreasing plasma insulin levels in a subject, and increasing skeletal muscle mass in a subject. Also provided are combinatorial treatments to bring about a weight reduction or reduction in obesity in a subject.

Before the present compositions and methods are described, it is to be understood that this invention is not limited to particular compositions, methods, and experimental conditions described, as such compositions, methods, and conditions may vary. It is also to be understood that the terminology used herein is for purposes of describing particular embodiments only, and is not intended to be limiting, since the scope of the present invention will be limited only in the appended claims.

As used in this specification and the appended claims, the singular forms "a", "an", and "the" include plural references unless the context clearly dictates otherwise. Thus, for example, references to "the method" includes one or more methods, and/or steps of the type described herein which will become apparent to those persons skilled in the art upon reading this disclosure and so forth.

Unless defined otherwise, all technical and scientific terms used herein have the same meaning as commonly understood by one of ordinary skill in the art to which this invention belongs. Although any methods and materials similar or equivalent to those described herein can be used in the practice or testing of the invention, the preferred methods and materials are now described.

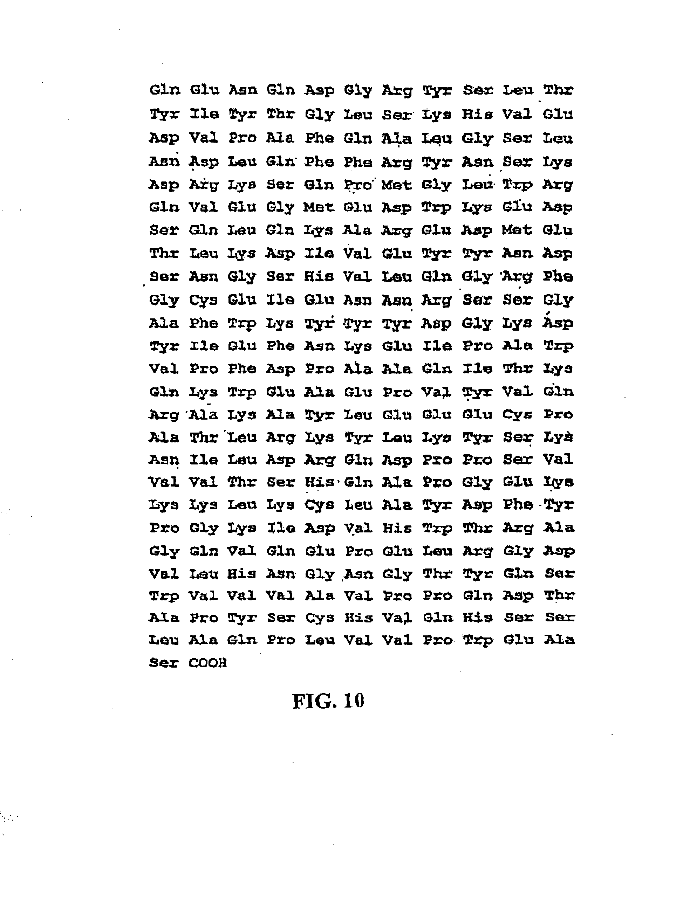

The complete amino acid sequence of ZAG has been reported in a paper entitled "Complete amino acid sequence of human plasma Zinc-α2-glycoprotein and its homology to histocompatibility antigens" by T. Araki et al. (1988) Proc. Natl. Acad. Sci. USA., 85, 679-683, wherein the glycoprotein was shown as consisting of a single polypeptide chain of 276 amino acid residues having three distinct domain structures (A, B and C) and including two disulfide bonds together with N-linked glycans at three glycosylation sites. This amino acid sequence of the polypeptide component is set out in Figure 10 of the accompanying drawings. Although some subsequent publications have indicated that the composition of human ZAG can vary somewhat when isolated from different body fluids or tissues, all preparations of this material have substantially the same immunological characteristics. As reported by H. Ueyama, et al. (1991) "Cloning and nucleotide sequence of a human Zinc-α2-glycoprotein cDNA and chromosomal assignment of its gene", Biochem. Biophys. Res. Commun. 177, 696-703, cDNA of ZAG has been isolated from human liver and prostate gland libraries, and also the gene has been isolated, as reported by Ueyama et al., (1993) "Molecular cloning and chromosomal assignment of the gene for human Zinc-α2-glycoprotein", Biochemistry 32, 12968-12976. H. Ueyama et al. have also described, in J. Biochem. (1994) 116, 677-681, studies on ZAG cDNAs from rat and mouse liver which, together with the glycoprotein expressed by the corresponding mRNAs, have been sequenced and compared with the human material. Although detail differences were found as would be expected from different species, a high degree of amino acid sequence homology was found with over 50% identity with the human counterpart (over 70% identity within domain B of the glycoprotein). Again, common immunological properties between the human, rat and mouse ZAG have been observed.

The purified ZAG discussed above was prepared from fresh human plasma substantially according to the method described by Ohkubo et al. (Ohkubo et al. (1988) "Purification and characterisation of human plasma Zn-α2-glycoprotein" Prep. Biochem., 18, 413-430). It will be appreciated that in some cases fragments of the isolated lipid mobilizing factor or of ZAG may be produced without loss of the lipolytic or lipid mobilizing activity, and various additions, deletions or substitutions may be made which also will not substantially affect this activity. As such, the methods of the invention also include use of functional fragments of ZAG. The glycoprotein or fragment thereof used in these therapeutic applications may further be produced by recombinant DNA techniques such as are well known in the art based possibly on the known cDNA sequence for Zn-α₂-glycoprotein which has been published for example in H. Ueyama et al. (1994) "Structure and Expression of Rat and Mouse mRNAs for Zn-α2-glycoprotein" J. Biochem., 116, 677-681. In addition, the glycoprotein or fragment thereof used in these therapeutic applications may further include post-expression modifications of the polypeptide, for example, glycosylations, acetylations, phosphorylations and the like, as well as other modifications known in the art, both naturally occurring and non-naturally occurring.

ZAG has been previously shown to bring about a weight reduction or reduction in obesity in mammals, as disclosed in U.S. Pat. No. 6,890,899, incorporated herein by reference in its entirety. In addition, it has been observed that a lipid mobilizing agent having similar characteristics of ZAG and/or fragments thereof has also been used to bring about a weight reduction or reduction in obesity in mammals, as disclosed in U.S. Published App. No.20060160723. The present invention demonstrates that ZAG and/or functional fragments thereof increases the insulin responsiveness of adipocytes and skeletal muscle, and produces an increase in muscle mass through an increase in protein synthesis coupled with a decrease in protein degradation regardless of whether a weight reduction or reduction in obesity is observed during treatment. It is therefore contemplated that the methods of the instant invention provide a detectable effect on symptoms associated with hyperglycemia and/or muscle wasting disease prior to any detectable weight reduction or reduction in obesity.

Accordingly, in one aspect, the disclosure provides a method of ameliorating symptoms of hyperglycemia in a subject. For example, the treatment regimen may be for months (e.g., 1, 2, 3, 4, 5, 6, 7, 8, 9, 10, 11, or 12 months), or years. The polypeptide is administered for a period of 21 days or longer. The amelioration of symptoms is detectable within days (*e.g.,* I, 2, 3, 4, 5, 6, or 7 days), weeks (*e.g*., 1, 2, 3, or 4 weeks), or months (e.g., 1, 2, 3, 4, 5, 6, 7, 8, 9, 10, 11, or 12 months) of initiating treatment. The treatment regimen may be about 21 days wherein there is amelioration of symptoms associated with hyperglycemia following treatment.

The term "subject" as used herein refers to any individual or patient to which the subject methods are performed. Generally the subject is human, although as will be appreciated by those in the art, the subject may be an animal. Thus other animals, including mammals such as rodents (including mice, rats, hamsters and guinea pigs), cats, dogs, rabbits, farm animals including cows, horses, goats, sheep, pigs, etc., and primates (including monkeys, chimpanzees, orangutans and gorillas) are included within the definition of subject.

Exemplary characterizations of hyperglycemia and/or disorders associated therewith include, but are not limited to, pre-diabetes, type 2 diabetes, type 1 diabetes, impaired glucose tolerance, polycystic ovarian syndrome, non alcoholic fatty liver disease, muscle wasting diseases, states of insulin resistance not producing detectable hyperglycemia, gestational diabetes, cardiovascular risk, and myocardial infarction. Exemplary muscle wasting diseases include, but are not limited to, cancer, AIDS, sepsis, COPD, renal failure, arthritis, congestive heart failure, muscular dystrophy, diabetes, sarcopenia of aging, severe trauma (e.g., orthopaedic immobilization of a limb), metabolic acidosis, denervation atrophy, and weightlessness. It should be understood that a subject having hyperglycemia and/or diabetes may be of normal or average body weight. Such individuals, referred to as "skinny diabetics" often exhibit one or more of the symptoms associated with hyperglycemia and/or diabetes without an increase in weight or obesity. In addition, a certain proportion of diabetics that are overweight or obese are, in fact, skinny diabetics with overweight/obesity superimposed on diabetes.

The term "therapeutically effective amount" or "effective amount" means the amount of a compound or pharmaceutical composition that will elicit the biological or medical response of a tissue, system, animal or human that is being sought by the researcher, veterinarian, medical doctor or other clinician.

The terms "administration" or "administering" are defined to include an act of providing a compound or pharmaceutical composition of the invention to a subject in need of treatment. The phrases "parenteral administration" and "administered parenterally" as used herein means modes of administration other than enteral and topical administration, usually orally or by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticulare, subcapsular, subarachnoid, intraspinal and intrasternal injection and infusion. The phrases "systemic administration," "administered systemically," "peripheral administration" and "administered peripherally" as used herein mean the administration of a compound, drug or other material other than directly into the central nervous system, such that it enters the subject's system and, thus, is subject to metabolism and other like processes, for example, subcutaneous administration.

As used herein, the term "ameliorating" or "treating" means that the clinical signs and/or the symptoms associated with hyperglycemia are lessened as a result of the actions performed. The signs or symptoms to be monitored will be characteristic of hyperglycemia and will be well known to the skilled clinician, as will the methods for monitoring the signs and conditions. Exemplary symptoms associated with hyperglycemia include, but are not limited to, increased serum levels of glucose, increased serum levels of triglycerides, increased serum levels of insulin and increased serum levels of non-esterified fatty acids (NEFA), as compared to a normal subject or a subject that does not have hyperglycemia. As such, an amelioration of the symptoms associate with hyperglycemia includes but is not limited to, decreasing serum levels of glucose, decreasing serum levels of triglycerides, decreasing serum levels of insulin, decreasing serum levels of non-esterified fatty acids, decreasing plasma insulin levels, increasing pancreatic insulin levels, and increasing skeletal muscle mass.

As used herein, the terms "reduce" and "inhibit" are used together because it is recognized that, in some cases, a decrease can be reduced below the level of detection of a particular assay. As such, it may not always be clear whether the expression level or activity is "reduced" below a level of detection of an assay, or is completely "inhibited". Nevertheless, it will be clearly determinable, following a treatment according to the present methods, that the level of, e.g., serum insulin, is at least reduced from the level prior to treatment.

ZAG has been attributed a number of biological roles, but its role as an adipokine regulating lipid mobilization and utilization is most important in regulating body composition. Previous studies suggested that the increase in protein synthesis was due to an increase in cyclic AMP through interaction with the β-adrenoreceptor, while the decrease in protein degradation was due to reduced activity of the ubiquitin-proteasome proteolytic pathway. Studies in db/db mice show that insulin resistance causes muscle wasting through an increased activity of the ubiquitin-proteasome pathway. An increased phosphorylation of both PKR and eIF2α will reduce protein synthesis by blocking translation initiation, while activation of PKR will increase protein degradation through activation of nuclear factor-κB (NF-κB), increasing expression of proteasome subunits. *In vitro* studies using myotubes in the presence of high extracellular glucose showed that activation of PKR led to activation of p38MAPK and formation of reactive oxygen species (ROS). p38MAPK can phosphorylate and activate cPLA₂ at Ser-505 causing release of arachidonic acid, a source of ROS. Hyperactivation of p38MAPK in skeletal muscle has been observed in models of diet-induced obesity. In addition caspase-3 activity has been shown to be increased in skeletal muscle of diabetic animals, which may be part of the signaling cascade, since it can cleave PKR leading to activation. Without being bound to theory, the ability of ZAG to attenuate these signaling pathways provides an explanation regarding its ability to increase muscle mass.

Accordingly, in another aspect, the disclosure provides a method of increasing skeletal muscle mass in a subject. The method includes administering to the subject a polypeptide having the sequence as shown in SEQ ID NO: 1.

In addition, ZAG has been shown to increase glucose oxidation and increase the tissue glucose metabolic rate in adult male mice. This increased utilization of glucose would explain the fall in both blood glucose and insulin levels in ob/ob mice administered ZAG. Triglyceride utilization was also increased in mice administered ZAG, which would explain the fall in plasma non-esterified fatty acids (NEFA) and triglycerides (TG) despite the increase in plasma glycerol, indicative of increased lipolysis. The increased utilization of lipid would be anticipated from the increased expression of UCP1 and UCP3 in BAT and UCP3 in skeletal muscle, resulting in an increase in body temperature. As such, in one option, amelioration of the symptoms associated with hyperglycemia also includes an increase in body temperature of about 0.5°C to about 1°C during treatment. In one option, the increase in body temperature occurs within 4 days of initiating treatment. In another option, amelioration of the symptoms associated with hyperglycemia also includes an increase in pancreatic insulin as compared to pancreatic insulin levels prior to treatment, since less insulin is needed to control blood glucose as a result of the presence of ZAG.

Thus, ZAG is identified as a lipid mobilizing factor capable of inducing lipolysis in white adipocytes of the mouse in a GTP-dependent process, similar to that induced by lipolytic hormones. The data presented herein supports these findings by showing that ZAG has a similar lipolytic effect in rat adipocytes, and, moreover, produces a decrease in body weight and carcass fat in mature male rats, despite the fact that the sequence homology between rat and human ZAG is only 59.4%.

ZAG also counters some of the metabolic features of the diabetic state including a reduction of plasma insulin levels and improved response in the glucose tolerance test. Thus, in another aspect, the disclosure provides a method of decreasing plasma insulin levels in a subject. The method includes administering to the subject a therapeutically effective dosage of a polypeptide having the sequence as shown in SEQ ID NO: 1. In one form, the decrease in plasma insulin occurs within 3 days of initiating treatment. The treatment regimen is administered for 21 days or longer.

In addition ZAG increases the responsiveness of epididymal adipocytes to the lipolytic effect of a β3-adrenergic stimulant. ZAG also increases the expression of HSL and ATGL in epididymal adipose tissue which have been found to be reduced in the obese insulin-resistant state. Factors regulating the expression of HSL and ATGL are not known. However, the specific ERK inhibitor, PD98059 downregulated HSL expression in response to ZAG, suggesting a role for MAPK in this process. Mice lacking MAPK phosphatase-1 have increase activities of ERK and p38MAPK in WAT, and are resistant to diet-induced obesity due to enhanced energy expenditure. Previous studies have suggested a role for MAPK in the ZAG-induced expression of UCP3 in skeletal muscle. ERK activation may regulate lipolysis in adipocytes by phosphorylation of serine residues of HSL, such as Ser-600, one of the sites phosphorylated by protein kinase A.

The results presented herein show that ZAG administration to the rat also increases the expression of ATGL and HSL in the rat. ATGL may be important in excess fat storage in obesity, since ATGL knockout mice have large fat deposits and reduced NEFA release from WAT in response to isoproterenol, although they did display normal insulin sensitivity. In contrast HSL null mice, when fed a normal diet, had body weights similar to wild-type animals. However, expression of both ATGL and HSL are reduced in human WAT in the obese insulin-resistant state compared with the insulin sensitive state, and weight reduction also decreased mRNA and protein levels.

As such, in one option, amelioration of the symptoms associated with hyperglycemia also includes an increase in expression of uncoupling protein-1 (UCP1) and uncoupling protein-3 (UCP3) in brown adipose tissue during treatment. In another option, amelioration of the symptoms associated with hyperglycemia also includes an increase in expression of UCP3 in skeletal muscle during treatment.

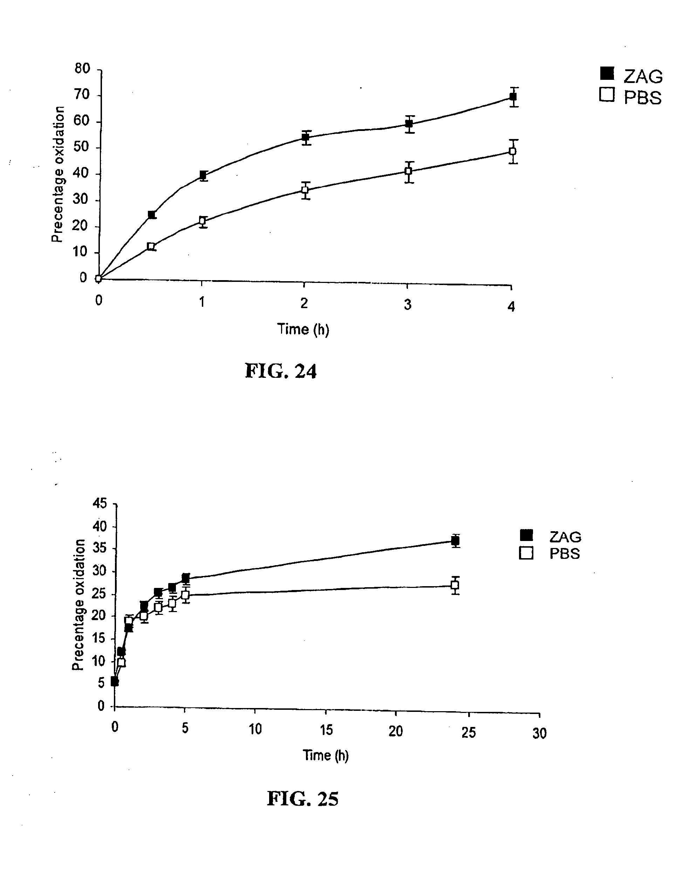

Stimulation of lipolysis alone would not deplete body fat stores, since without an energy sink the liberated NEFA would be resynthesised back into triglycerides in adipocytes. To reduce body fat, ZAG not only increases lipolysis, as shown by an increase in plasma glycerol, but also increases lipid utilization, as shown by the decrease in plasma levels of triglycerides and NEFA. This energy is channeled into heat, as evidenced by the 0.4°C rise in body temperature in rats treated with ZAG. The increased energy utilization most likely arises from the increased expression of UCP1, which has been shown in both BAT and WAT after administration of ZAG. An increased expression of UCP1 would be expected to decrease plasma levels of NEFA, since they are the primary substrates for thermogenesis in BAT. BAT also has a high capacity for glucose utilization, which could partially explain the decrease in blood glucose. In addition there was increased expression of GLUT4 in skeletal muscle and WAT, which helps mediate the increase in glucose uptake in the presence of insulin. In mice treated with ZAG there was an increased glucose utilization/oxidation by brain, heart, BAT and gastrocnemius muscle, and increased production of ¹⁴CO₂ from D-[U-¹⁴C] glucose, as well as [¹⁴C carboxy] triolein (Figure 24). There was also a three-fold increase in oxygen uptake by BAT of ob/ob mice after ZAG administration.

While ZAG increased expression of HSL in epididymal adipocytes there was no increase in either subcutaneous or visceral adipocytes. A similar situation was observed with expression of adipose triglyceride lipase (ATGL). Expression of HSL and ATGL correlated with expression of the active (phospho) form of ERK. Expression of HSL and ATGL in epididymal adipocytes correlated with an increased lipolytic response to the β3 agonist, BRL37344. This result suggests that ZAG may act synergistically with β3 agonists.

As used herein, the term "agonist" refers to an agent or analog that is capable of inducing a full or partial pharmacological response. For example, an agonist may bind productively to a receptor and mimic the physiological reaction thereto. As such, the methods of the invention may further include administering ZAG, or a fragment thereof, in combination with a β3 agonist such as BRL37344.

Induction of lipolysis in rat adipocytes by ZAG is suggested to be mediated through a β3-AR, and the effect of ZAG on adipose tissue and lean body mass may also be due to its ability to stimulate the β3-AR. Induction of UCP1 expression by ZAG has been shown to be mediated through interaction with a β3-AR. The increased expression of UCP1 in WAT may also be a β3-AR effect through remodeling of brown adipocyte precursors, as occurs with the β3-AR agonist CL316,243. Using knock-out mice the antiobesity effect of β3-AR stimulation has been mainly attributed to UCP1 in BAT, and less to UCP2 and UCP3 through the UCP1-dependent degradation of NEFA released from WAT. Glucose uptake into peripheral tissues of animals is stimulated by cold-exposure, an effect also mediated through the β3-AR. However, targeting the β3-AR has been more difficult in humans than in rodents, since β3-AR play a less prominent role than β1 and β2-AR subtypes in the control of lipolysis and nutritive blood flow in human subcutaneous abdominal adipose tissue. However, despite this the β3-AR agonist CL316,243 has been shown to increase fat oxidation in healthy young male volunteers. This may be due to the ability of β-adrenergic agonists to increase the number of β3-AR in plasma membranes from BAT.

Recent results suggest that ZAG expression in adipose tissue may be more important locally than circulating ZAG, by acting in a paracrine manner. Thus in humans, while mRNA levels of ZAG in visceral and subcutaneous fat correlated negatively with BMI, fat mass and insulin resistance, serum levels, determined by ELISA, correlated positively with parameters of adiposity (BMI and waist circumference) and insulin resistance. Thus the ability of ZAG to induce its own expression in gastrocnemius muscle, WAT and BAT may be critical for its ability to increase lipolysis and energy utilization.

These results provide evidence for the ability of ZAG to mobilize and utilize lipid in rats, confirming a species independent effect, and suggest that it may be useful as an antiobesity agent in man. Accordingly, in another aspect, the disclosure provides a method of treating a subject to bring about a weight reduction or reduction in obesity. The method includes administering to the subject in need of such treatment a therapeutically effective dosage of a β3 agonist in combination with a polypeptide having the sequence as shown in SEQ ID NO: 1. In another option the method of treating a subject to bring about a weight reduction or reduction in obesity includes administering to the subject in need of such treatment a therapeutically effective dosage of a β3-AR antagonist in combination with a polypeptide having the sequence as shown in SEQ ID NO: 1.

All methods may further include the step of bringing the active ingredient(s) (e.g., ZAG or functional fragments of ZAG) into association with a pharmaceutically acceptable carrier, which constitutes one or more accessory ingredients. As such, the invention also provides pharmaceutical compositions for use in treating subjects having symptoms associated with hyperglycemia. The composition includes as the active constituent a therapeutically effective amount of ZAG or glycoprotein lipid mobilizing factor as discussed above, together with a pharmaceutically acceptable carrier, diluent of excipient.

Pharmaceutically acceptable carriers useful for formulating a composition for administration to a subject are well known in the art and include, for example, aqueous solutions such as water or physiologically buffered saline or other solvents or vehicles such as glycols, glycerol, oils such as olive oil or injectable organic esters. A pharmaceutically acceptable carrier can contain physiologically acceptable compounds that act, for example, to stabilize or to increase the absorption of the conjugate. Such physiologically acceptable compounds include, for example, carbohydrates, such as glucose, sucrose or dextrans, antioxidants, such as ascorbic acid or glutathione, chelating agents, low molecular weight proteins or other stabilizers or excipients. One skilled in the art would know that the choice of a pharmaceutically acceptable carrier, including a physiologically acceptable compound, depends, for example, on the physico-chemical characteristics of the therapeutic agent and on the route of administration of the composition, which can be, for example, orally or parenterally such as intravenously, and by injection, intubation, or other such method known in the art. The pharmaceutical composition also can contain a second (or more) compound(s) such as a diagnostic reagent, nutritional substance, toxin, or therapeutic agent, for example, a cancer chemotherapeutic agent and/or vitamin(s).

Formulations of the present invention suitable for oral administration may be presented as discrete units such as capsules, cachets, tablets or lozenges, each containing a predetermined amount of the active compound in the form of a powder or granules; or as a suspension of the active compound in an aqueous liquid or non-aqueous liquid such as a syrup, an elixir, an emulsion of a draught.

Accordingly, in another aspect, the disclosure provides the use of ZAG or a lipid mobilizing agent, as herein defined, for the manufacture of a medicament useful in human medicine for treating symptoms and/or conditions associated with hyperglycemia. Such a medicament may also be useful for stimulating muscle development and increasing muscle mass, decreasing serum levels of glucose, decreasing serum levels of triglycerides, decreasing serum levels of insulin, decreasing serum levels of non-esterified fatty acids, decreasing plasma insulin levels, and/or increasing pancreatic insulin levels.

The total amount of a compound or composition to be administered in practicing a method of the disclosure can be administered to a subject as a single dose, either as a bolus or by infusion over a relatively short period of time, or can be administered using a fractionated treatment protocol, in which multiple doses are administered over a prolonged period of time (*e.g*., once daily, twice daily, etc.). One skilled in the art would know that the amount of ZAG or functional fragment thereof to treat symptoms associated with hyperglycemia in a subject depends on many factors including the age and general health of the subject as well as the route of administration and the number of treatments to be administered. In view of these factors, the skilled artisan would adjust the particular dose as necessary. In general, the formulation of the pharmaceutical composition and the routes and frequency of administration are determined, initially, using Phase I and Phase II clinical trials.

Accordingly, in certain options, the methods of the disclosure include an intervalled treatment regimen. It was observed that long-term daily administration of ZAG in ob/ob mice results in a cessation of weight loss. As such in one option, the treatment is administered every other day. In another option, the treatment is administered every two days. In another option, the treatment is administered every three days. In another option, the treatment is administered every four days.

Thus, the invention demonstrates that additional weight loss and/or amelioration of the symptoms associated with hyperglycemia occurs when administration of ZAG is interrupted for approximately 3-4 days followed by re-infusion. Without being bound to theory, this may be because too much is being administered or that there is receptor desensitization as is seen with TNF. A pilot study with 2 mice in each group was performed and an 8-10g weight loss from a 90g mouse was observed in about 3 weeks. As such, it is contemplated that smaller dosages of ZAG or a functional fragment thereof may be used to ameliorate the symptoms of hyperglycemia in a subject prior to an observable decrease in obesity or reduction in weight.

The following examples are provided to further illustrate the advantages and features of the present invention, but are not intended to limit the scope of the invention. While they are typical of those that might be used, other procedures, methodologies, or techniques known to those skilled in the art may alternatively be used.

### EXAMPLE 1

### Zinc-α₂-glycoprotein attenuates hyperglycemia

To evaluate the ability of Zinc-α₂-glycoprotein (ZAG) to attenuate obesity and hyperglycemia ob/ob mice were administered ZAG which induced a loss of body weight, and a rise in body temperature, suggesting an increased energy expenditure. Expression of uncoupling proteins-1 and -3 in brown adipose tissue were increased, while there was a decrease in serum levels of glucose, triglycerides and non-esterified fatty acids, despite an increase in glycerol, indicative of increased lipolysis. There was a decrease in plasma insulin and an improved response to intravenous glucose together with an increased glucose uptake into adipocytes and skeletal muscle. Expression of hormone-sensitive lipase in epididymal adipocytes was increased. There was an increase in skeletal muscle mass due to an increase in protein synthesis and decrease in degradation. This suggests that ZAG may be effective in the treatment of hyperglycemia.

Dulbeccos' Modified Eagle's (DMEM) and Freestyle media were purchased from Invitrogen (Paisley, UK) while fetal calf serum was from Biosera (Sussex, UK). 2-[1-¹⁴C] Deoxy-D-glucose (sp.act.1.85GBq mmol⁻¹) and L-[2,6-³H] phenylalanine (sp.act.37Bq mmol⁻¹) were from American Radiolabeled Chemicals (Cardiff, UK). Rabbit polyclonal antibody to phospho (Thr-202) and total ERK1, total p38MAPK, phospho HSL (Ser-552), glucose transporter 4 (GLUT4), adipose triglyceride lipase, hormone sensitive lipase, and phospho PLA₂ (Ser-505) and to human ATGL were purchased from Abcam (Cambridge, UK). Mouse monoclonal antibody to full length human ZAG was from Santa Cruz (California, USA), and mouse monoclonal antibody to myosin heavy chain type II was from Novacastra (via Leica Biosystems, Newcastle, UK). Mouse monoclonal antibodies to 20S proteasome α-subunits and p42 were from Affiniti Research Products (Exeter, UK). Mouse monoclonal antibody to phospho (Thr-180/Tyr-182) p38MAPK and rabbit polyclonal antisera to total and phospho (Thr-451) PKR, phospho (Ser-162) eIF2α and to total eIF2α were from New England Biosciences (Herts, UK). Polyclonal rabbit antibodies to UCP1, UCP3 and total PKR and PHOSPHOSAFE™ Extraction Reagent were from Calbiochem (via Merk Chemicals, Nottingham, UK). Peroxidase-conjugated goat anti-rabbit and rabbit anti-mouse antibodies were purchased from Dako (Cambridge, UK). Polyclonal rabbit antibody to mouse β-actin and the triglyceride assay kit were purchased from Sigma Aldrich (Dorset, UK). Hybond A nitrocellulose membranes and enhanced chemiluminescence (ECL) development kits were from Amersham Pharmacia Biotech (Bucks, UK). A WAKO colorimetric assay kit for NEFA was purchased from Alpha Laboratories (Hampshire, UK), and a mouse insulin ELISA kit was purchased from DRG (Marburg, Germany). Glucose measurements were made using a Boots (Nottingham, UK) plasma glucose kit.

*Production of recombinant Z4G -* HEK293F cells were transfected with full length human ZAG cDNA in the expression vector pcDNA 3.1, and maintained in FreeStyle medium under an atmosphere of 5% CO₂ in air at 37°C. ZAG was secreted into the medium, which was collected, and maximal protein levels (16µgml⁻¹) were obtained after 14 days of culture. To purify ZAG, media (200ml) was centrifuged at 700g for 15min to remove cells, and concentrated into a volume of 1ml sterile PBS using an Amicon Ultra-15 centrifugal filter with a 10kDa cut-off. The concentrate (about 2mg protein) was added to 2g DEAE cellulose suspended in 20ml 10mM Tris, pH 8.8 and stirred for 2h at 4°C. The DEAE cellulose bound ZAG and it was sedimented by centrifugation (1500g for 15min) and the ZAG was eluted by stirring with 20ml 10mM Tris, pH8.8 containing 0.3M NaCl for 30min at 4°C. The eluate was washed and concentrated into a volume of 1ml in sterile PBS using an Amicon centrifugal filter. The purified ZAG was free of endotoxin, as determined with a LAL Pyrogent single test kit (Lonza, Bucks, UK).

*Cell culture and purification of ZAG.* Single-cell suspensions of white adipocytes were prepared from minced adipose deposits by incubation at 37°C for 2h in Krebs-Ringer biocarbonate buffer containing 1.5mgml⁻¹ collagenase, and 4% bovine serum albumin under an atmosphere of 95% oxygen : 5% CO₂ as previously described. For time-course studies adipocytes were suspended in DMEM containing 10% fetal calf serum at a concentration of 10⁵ cells ml⁻¹ and maintained under an atmosphere of 10% CO₂ in air at 37°C. Human 293 cells transfected with a plasmid containing human ZAG were seeded at a concentration of 10⁵ cells ml⁻¹ in FreeStyle medium and maintained under an atmosphere of 5% CO₂ in air at 37°C. Maximal protein levels (16µgm⁻¹) were obtained after 14 days of culture. The media (200ml) was then centrifuged at 700g for 15min to remove cells and concentrated into a volume of 1ml of sterile PBS using an Amicon Ultra-15 centrifugal filter with a 10kDa cutoff. After measurement of the protein concentration of the sample (about 2mg) it was added to 2g DEAE cellulose suspended in 20ml of 10mM Tris, pH8.8 and stirred at 4°C for 2h. ZAG being negatively charged binds to the DEAE cellulose, which was sedimented by centrifugation (1500g for 15min), and eluted by stirring with 20ml 10mM Tris, pH8.8 containing 0.3M NaCl for 30min at 4°C. The supernatant was washed and concentrated to a volume of 1ml in sterile PBS using the Amicon centrifugal filter.

*Animals - mice.* Homozygous obese (ob/ob) mice from the colony maintained at Aston University were used in the present study. The origin and characteristics of Aston ob/ob mice have been previously described. Male mice (20-21 weeks old, weight 90-100g) were grouped into three per cage in an air conditioned room at 22 ± 2°C with a 12h-light:12h-dark cycle and fed a rat and mouse breeding diet (Special Diet Services, Witham, UK) and tap water ad libitum. They were administered ZAG (35µg) in PBS (100µl) b.d. by i.v. administration and body weight and food and water intake were monitored daily. Control mice received PBS alone. Body temperature was measured daily by the use of a rectal thermometer (RS Components, Northants, UK). All animal experiments were carried out in accordance with the U.K. Animals (Scientific Procedures) Act 1986. No adverse effects were observed after administration of ZAG.

*Animal - Rats.* Mature male Wistar rats (one year old from our own colony) weighing 540±82.5g were housed individually and treated once daily i.v., with either ZAG in PBS (100µl) (50µg per 100g body weight), or with PBS (100µl) as a control. Both food and water intake and body weight were measured daily. Animals were given free access to food (Special Diet Services, Essex, UK) and water ad libitum. The animal experiment was carried out under the welfare conditions imposed by the British Home Office. After 10 days treatment the animals were terminated and the body composition determined. Animals were heated to 80-90°C for 7 days until constant weight was achieved. The water content was then determined from the difference between the wet and dry weight. Lipids were extracted from the dry carcass using a sequence of chloroform:methanol (1:1), ethanol/acetone (1:1) and diethyl ether (120ml of each) as described by Lundholm *et al* (14). The solvents were evaporated and the fat weighed. The non-fat carcass mass was calculated as the difference between the initial weight of the carcass and the weight of water and fat.

*Lipolytic assay.* Samples to be assayed were incubated with 10⁵ to 2x10⁵ adipocytes for 2h in 1ml Krebs-Ringer bicarbonate buffer, pH 7.2. The concentration of glycerol released was determined enzymatically by the method of Wieland (Wieland, O. Glycerol UV method. In Methods of Enzymatic Analysis (ed. Bergmeyer, H.U.) (Academic Press, London, UK, pp 1404-1409, 1974)). Control samples containing adipocytes alone were analysed to determine the spontaneous glycerol release. Activity was expressed as µmol glycerol released/10⁵ adipocytes/2h.

*Serum Metabolite Determinations.* Non-esterified fatty acids (NEFA) were determined using a Wako-ASC-ACOD kit (Wako Chemical GmbH, Neuss, Germany). Triglycerides were determined using a Triglyceride kit (Sigma Chemical Co., Poole, United Kingdom) and 3-hydroxybutyrate by a quantitative enzymatic determination kit (Sigma). Glucose was measured using a glucose analyser (Beckman, Irvine, Calif.) and glycerol was determined enzymatically using the method of Wieland as described in "Methods of Enzymatic Analysis" (Ed. Bergmeyer, H. U.) Vol. 3, pp1404-1409, published by Academic Press, London (1974).

*Isolation of Mouse Adipocyte Plasma Membranes.* In a typical procedure white adipocytes were isolated from mouse epididymal fat pads as referred to above except that the cells were washed in 250 mM sucrose, 2 mM ethyleneglycol bis(β-aminoethylether)-N,N,N',N' (EGTA), 10 mM Tris-HCl (pH 7.4). Adipocytes were resuspended in 20 ml of the above buffer and homogenised by aspirating through a Swinny filter at least 10 times. The cell homogenate was then centrifuged at 300 g for 5 min, the fat cake removed from the surface and the remaining pellet and infranatant transferred to clean tubes. These were centrifuged at 30,000 g for 1 h at 4°C and the membrane pellet formed was resuspended in the sucrose buffer (200 to 400 µl). Plasma membranes were separated from other organelle membranes on a self-forming gradient of PERCOLL™ colloidial silica particles. The constituents were 250 mM sucrose, 2 mM EGTA, 10 mM Tris-HCl, pH 7.4; PERCOLL™; and 2M sucrose, 8 mM EGTA, 80 mM Tris-HCl, pH 7.4, mixed in a ratio of 32:7:1 together with the membrane suspension (in a total volume of 8 ml). This mixture was centrifuged at 10,000 g for 30 min at 4°C. The gradient was fractionated into 0.75 ml portions and each portion was assayed for the presence of succinate dehydrogenase, NADH-cytochrome c reductase, lactate dehydrogenase and 5'-nucleotidase to locate the plasma membrane fraction. The membrane fractions were resuspended in 150 mM NaCl, 1 mM EGTA, 10 mM Tris-HCl, pH 7.4 and centrifuged at 10,000 g at 4°C for 2 min. The process was repeated twice. The washed plasma membranes were then diluted in 10 mM Tris-HCl, pH 7.4, 250 mM sucrose, 2 mM EGTA and 4 µM phenylmethylsulfonyl fluoride (PMSF) at 1-2 mg/ml, snap frozen in liquid nitrogen and stored at -70°C until use.

*Lipolytic activity in rat adipocytes -* White adipocytes were prepared from finely minced epididymal adipose tissue of male Wistar rats (400g) using collagenase digestion, as described (Beck SA, et al. Production of lipolytic and proteolytic factors by a murine tumor-producing cachexia in the host. Cancer Res 47:5919-5923, 1987). Lipolytic activity was determined by incubating 10⁵-2x10⁵ adipocytes for 2h in 1ml Krebs-Ringer bicarbonate buffer, pH 7.2, and the extent of lipolysis was determined by measuring the glycerol released (Wieland O. Glycerol UV method. Methods of Enzymatic Analysis, edited by Bergmeyer HU. Academic Press, London, pp 1404-1409, 1974). Spontaneous glycerol release was measured by incubating adipocytes alone. Lipolytic activity was expressed as µmol glycerol released/10⁵ adipocytes/2h.

*Gel Electrophoresis.* Gels were prepared according to the method of Laemmli and generally consisted of a 5% stacking gel and a 15% SDS-PAGE resolving gel (denaturing or reducing conditions) or a 10% SDS-PAGE resolving gel (non-denaturing or non-reducing conditions). Samples were loaded at 1-5 µg/lane. Bands were visualized by staining either with Coomassie brilliant blue R-250 or by silver. Samples were prepared for reducing conditions by heating for 5 min at 100°C in 0.0625M Tris-HCl, pH 6.8, 10% glycerol, 1% SDS, 0.01% bromophenol blue and 5% 2-mercaptoethanol.

*Glucose uptake into adipocytyes.* Isolated adipocytes (5x10⁴) were washed twice in 1 ml Krebs-Ringer bicarbonate buffer, pH 7.2 (KRBS) and further incubated for 10min at room temperature in 0.5ml KRBS containing 18.5MBq 2-[1-¹⁴C] deoxy-D-glucose and non-radioactive 2-deoxy-D-glucose to a final concentration of 0.1mM. Uptake was terminated by the addition of 1ml ice-cold glucose-free KRBS, and the cells were washed three times with 1ml KRBS, lysed by addition of 0.5ml 1M NaOH and left for at least 1h at room temperature before the radioactivity was determined by liquid scintillation counting.

*Glucose uptake into gastrocnemius muscle -* Gastrocnemius muscles were incubated in Krebs-Henseleit bicarbonate buffer for 45min at 37°C and then incubated for a further 10min in 5ml Krebs-Henseleit buffer containing 185M Bq 2-[1-¹⁴C] deoxy-D-glucose and non-radioactive 2-deoxy-D-glucose to a final concentration of 0.1mM. The muscles were then removed and washed in 0.9% NaCl for 5min followed by dissolution in 0.5ml 1M NaOH and the radioactivity was determined by liquid scintillation counting.

*Glucose uptake into soleus muscle.* Soleus muscles were incubated in Krebs-Henseleit bicarbonate buffer for 45min at 37°C and then incubated for a further 10min in 5ml Krebs-Henseleit buffer containing 185MBq 2-[1-¹⁴C] deoxy-D-glucose and non-radioactive 2-deoxy-D-glucose to a final concentration of 0.1mM. The muscles were then removed and washed in 0.9% NaCl for 5 min, followed by dissolution in 0.5ml 1MNaOH and the radioactivity was determined by liquid scintillation counting

*Protein synthesis and degradation in muscle.* The method for the determination of protein synthesis and degradation in muscle has been previously described (Smith, K.L. & Tisdale, M.J. Increased protein degradation and decreased protein synthesis in skeletal muscle during cancer cachexia. Br. J. Cancer 67, 680-685 (1993)). Gastrocnemius muscles were excised using ligatures and incubated for 30 min at 37°C in RPMI 1640 medium lacking phenol red and saturated with O₂:CO₂ (19:1) and then washed with PBS. Protein synthesis was measured by the incorporation of L-[2,6-³H] phenylalanine (640 MB^{q}) into acid-insoluble material using a 2h period in which the muscles were incubated at 37°C in RPMI/640 without phenol red and saturated with O₂:CO₂ (19:1). Muscles were then rinsed in non-radioactive medium, blotted and homogenised in 2% perchloric acid. The rate of protein synthesis was calculated by dividing the amount of protein-bound radioactivity by the amount of acid soluble radioactivity. Protein degradation was determined by the release of tyrosine from gastrocnemius muscle over a 2h period in 3ml of oxygenated Krebs-Henseleit buffer, pH7.4, containing 5mM glucose and 0.5mM cycloheximide.

*Measurement of proteasome and caspase activity.* The 'chymotrypsin-like' activity of the proteasome was determined fluorometrically by measuring the release of 7-amido-4-methylcoumarin (AMC) at an excitation wavelength of 360nm and an emission wavelength of 460nm from the fluorogenic substrate N-succinyl Lys Lys Val Tyr.AMC (SEQ ID NO: 2) as previously described for myotubes (Whitehouse, A.S. & Tisdale, M.J. Increased expression of the ubiquitin-proteasome pathway in murine myotubes by proteolysis-inducing factor (PIF) is associated with activation of the transcription factor NF-κB. Br. J. Cancer 89, 1116-1122 (2003)). Gastrocnemius muscle was homogenised in 20mM Tris, pH7.5, 2mM ATP, 5mM MgCl₂ and 50mM DTT at 4°C, sonicated and centrifuged at 18,000g for 10min at 4°C to pellet insoluble material, and the resulting supernatant was used to measure 'chymotrypsin-like' enzyme activity in the presence or absence of the proteasome inhibitor lactacystin (10µM). Only lactacystin suppressible activity was considered as true proteasome activity. The activity of caspase-3 was determined by the release of AMC from AcAsp.Gly.Val.Asp.AMC (SEQ ID NO: 3), and the activity of caspase-8 was determined by the release of 7-amino-4-trifluromethylcoumarin (AFC) from the specific substrate Z-Ile Glu Phe Thr Asp-AFC (SEQ ID NO: 4), using the supernatant from above (50µg protein), and either the caspase-3 or -8 substrate (10µM) for 1h at 37°C, in the presence or absence of the caspase-3 (AcAspGluValAsp-CHO) (SEQ ID NO: 5) or caspase-8 (Ile Glu Phe Thr Asp-CHO) (SEQ ID NO: 6) inhibitors (100µM). The increase in fluorescence due to AFC was determined as above, while the increase in fluorescence due to AFC was measured with an excitation wavelength of 400nm and an emission wavelength of 505nm. The difference in values in the absence and presence of the caspase inhibitors was a measure of activity.

*Western blot analysis.* Freshly excised gastrocnemius muscles were washed in PBS and lysed in PHOSPHOSAFE™ Extraction Reagent for 5min at room temperature followed by sonication at 4°C. The lysate was cleared by centrifugation at 18,000g for 5min at 4°C and samples of cytosolic protein (5µg) were resolved on 12% sodium dodecyl suflate-polyacrylamide gel electrophoresis at 180V for approximately 1h. This was followed by transference to 0.45µm nitrocellulose membranes, which were then blocked with 5% Marvel in Tris-buffered saline, pH 7.5, at 4°C overnight. Both primary and secondary antibodies were used at a dilution of 1:1000 except anti-myosin (1:250). Incubation was for 1h at room temperature, and development was by ECL. Blots were scanned by a densitometer to quantify differences.

Samples of epididymal WAT, BAT and gastrocnemius muscle excised from rats treated with ZAG or PBS for 5 days were homogenized in 0.25M sucrose, 1mM HEPES, pH 7.0 and 0.2M EDTA, and then centrifuged for 10min at 4,500 rpm. Samples of cytosolic protein (10µg) were resolved on 12% sodium dodecylsulphate polyacrylamide gel electrophoresis and the proteins were then transferred onto 0.45µm nitrocellulose membranes, which had been blocked with 5% Marvel in Tris-buffered saline, pH 7.5, at 4°C overnight, and following four 15 min washes with 0.1% Tween in PBS, incubation with the secondary antibody was performed for 1h at room temperature. Development was by ECL.

*Statistical analysis.* The results are shown as means ± SEM for at least three replicate experiments. Difference in means between groups was determined by one-way analysis of variance (ANOVA) followed by the Tukey-Kramer multiple comparison test. P values less than 0.05 were considered significant.

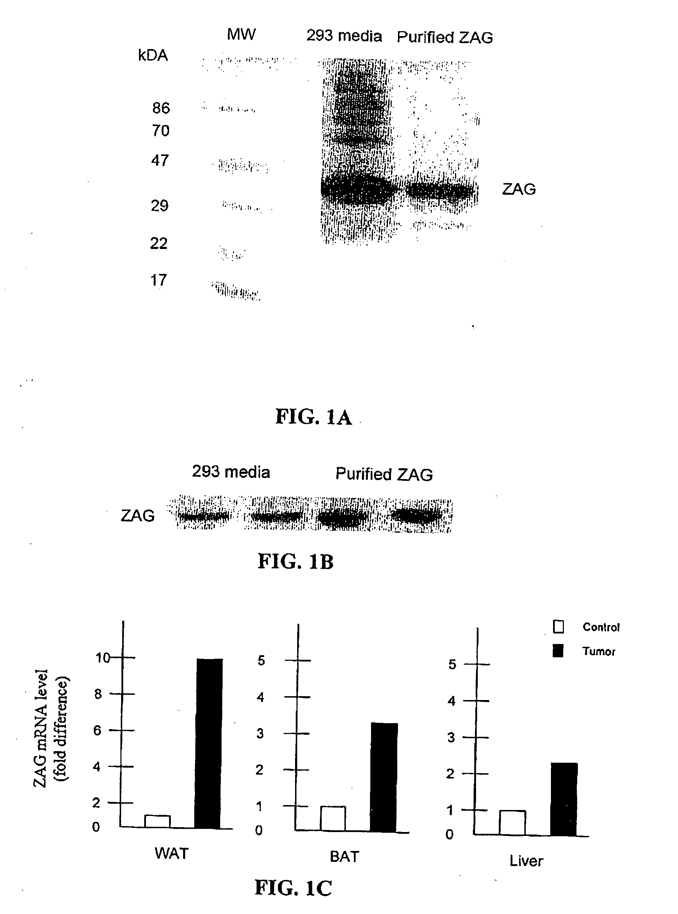

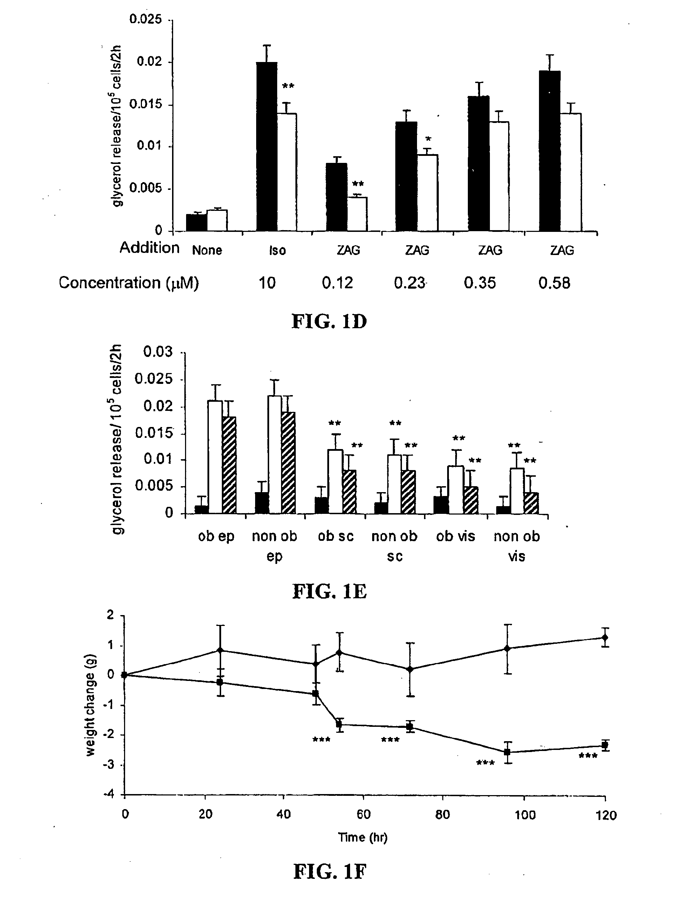

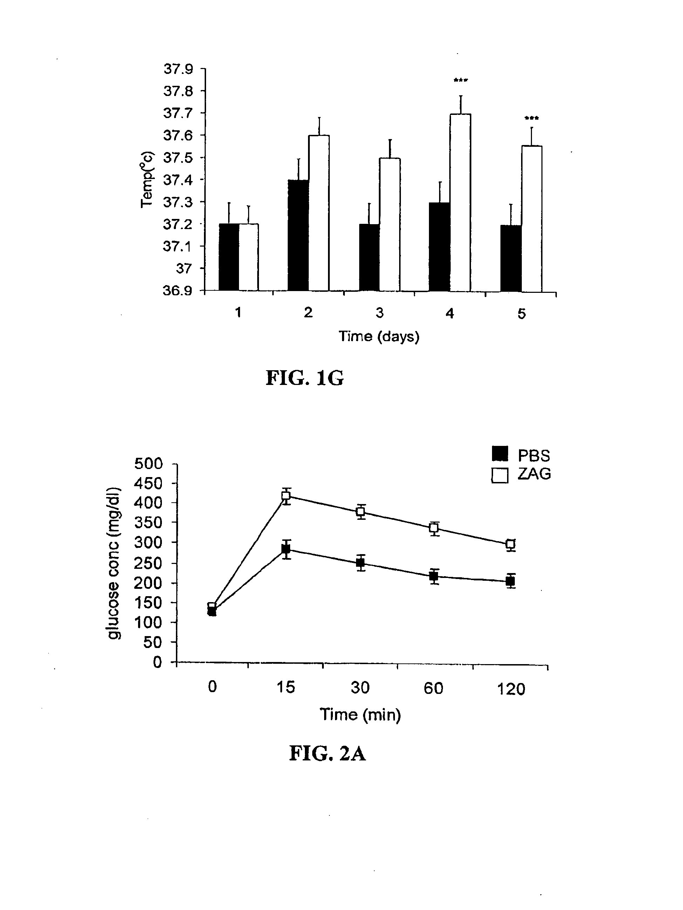

*Results - mice.* Purification of ZAG resulted in a product that was greater than 95% pure (Figure 1A), confirmed as ZAG by immunoblotting (Figure 1B). ZAG stimulated lipolysis in epididymal adipocytes (Figure 1D) but the lipolytic effect was considerably reduced in adipocytes from both subcutaneous and visceral deposits, although it was significantly elevated over basal levels (Figure 1E). There was no significant difference in the extent of stimulation of lipolysis between isoprenaline and ZAG in any adipocyte group, although ZAG was more potent at inducing lipolysis than isoprenaline on a molar basis. The effect of ZAG on the body weight of ob/ob mice over a 5 day period is shown in Figure 1F. While control animals remained weight stable, animals treated with ZAG showed a progressive weight loss, such that after 5 days there was a 3.5g weight difference between the groups, despite equal food (PBS 32 ± 3.1g; ZAG 30 ± 2.5g) and water (PBS 140 ± 8.2ml; ZAG 135 ± 3.2ml) intake over the course of the experiment. There was a significant rise of body temperature of 0.4°C after 4 days of ZAG administration (Figure 1G), indicative of an increase in basal metabolic rate. Measurement of plasma metabolite levels suggest an increase in metabolic substrate utilization in ZAG treated animals (Table 1). Thus there was a significant decrease in plasma glucose, triglycerides (TG) and non-esterified fatty acids (NEFA) in ZAG-treated animals, despite an increased glycerol concentration indicative of an increased lipolysis. There was a 36% decrease in plasma insulin levels suggesting that ZAG is effective in reducing the diabetic state. ZAG mRNA levels in various tissues are shown in Figure 1C.

**Table 1 - Plasma metabolite and insulin levels in ob/ob mice treated with ZAG for 120h**

| | **PBS** | **ZAG** |
|---|---|---|
| Glucose (mmol/L) | 24.5 + 0.4 | 20.3 + 0.8 p<0.01 |
| TG (mmol/L) | 1.2 + 0.3 | 0.9 + 0.1 p<0.05 |
| Glycerol (µmol/L) | 359 + 23 | 429 + 36 p<0.001 |
| Insulin (ng/mL) | 41.2 + 0.6 | 26.3 + 0.52 P<0.001 |
| BAT (g) | 0.35 ± 0.09 | 0.73 ± 0.12 p<0.01 |
| NEFA (mEq/L) | 0.6 + 0.12 | 0.23 + 0.05 p<0.001 |
| Soleus (g) | 0.52 ± 0.13 | 0.80 ± 0.09 p<0.01 |
| Gastrocnemius (g) | 0.85 ± 0.12 | 1.12 ± 0.14 p<0.01 |
| Insulin Pancreas (pg/g pancerase) | 4.52 ± 2.91 | 16.3 ± 3.1 p=0.0042 |

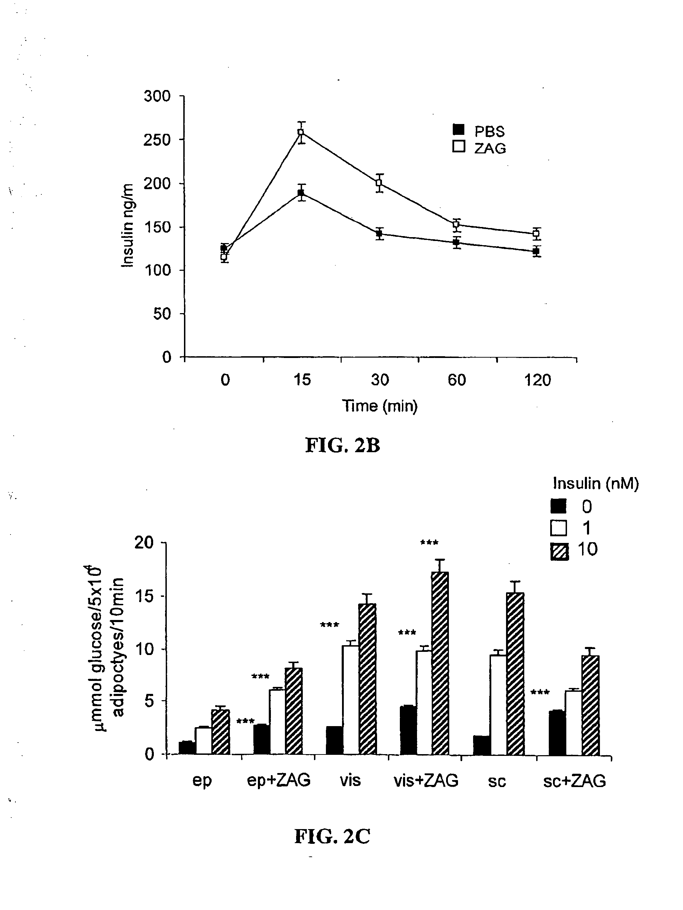

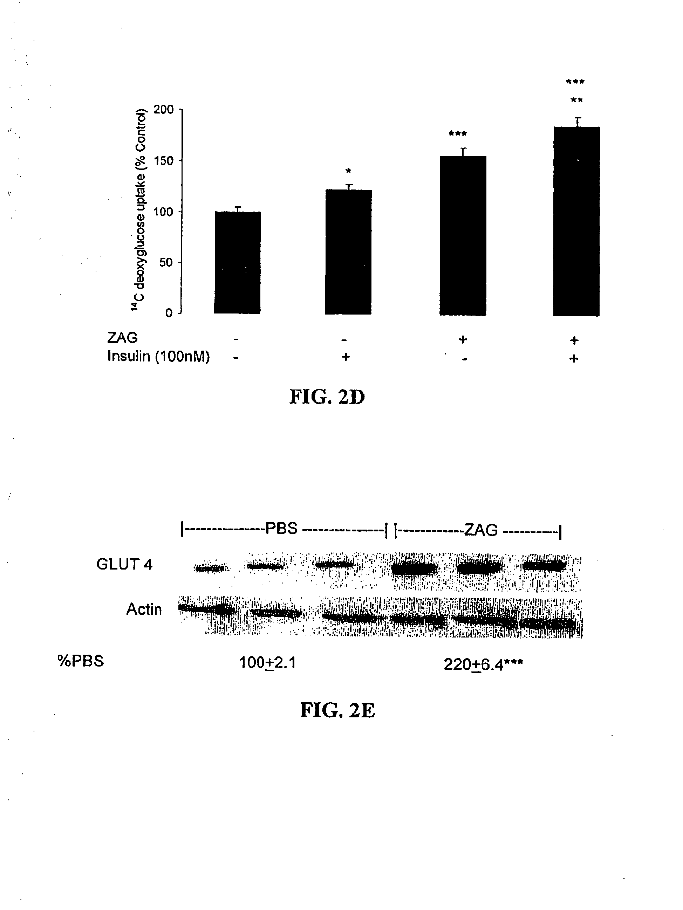

To investigate this, a glucose tolerance test was performed, on fed animals, after 3 days of ZAG administration (Figure 2A). While blood glucose levels were significantly elevated in PBS controls, there was only a small rise in ZAG treated animals, which remained significantly below the control group throughout the course of the study. In addition plasma insulin levels were significantly lower in ZAG treated animals at the onset of the study and remained so during the 60min of observation (Figure 2B). ZAG administration increased glucose uptake into epididymal, visceral and subcutaneous adipocytes in the absence of insulin and also increased glucose uptake into epididymal and visceral adipocytes in the presence of low (1nM) insulin (Figure 2C). Glucose uptake into gastrocnemius muscle was also significantly enhanced in ZAG treated animals both in the absence and presence of insulin (100nM) (Figure 2D). The glucose uptake in gastrocnemius muscle of ZAG treated mice was greater than the response to insulin in non-treated animals.

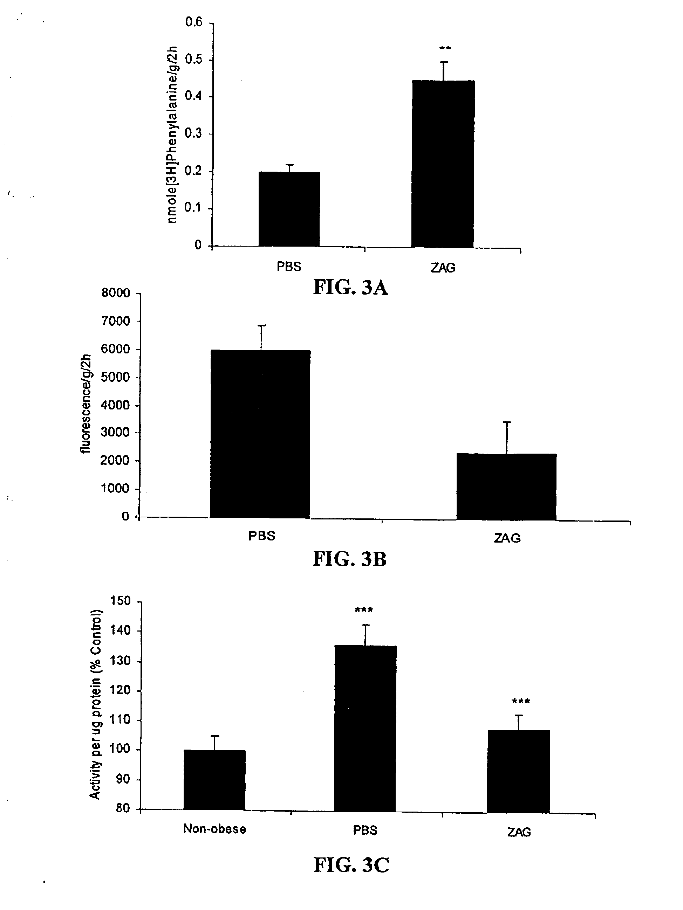

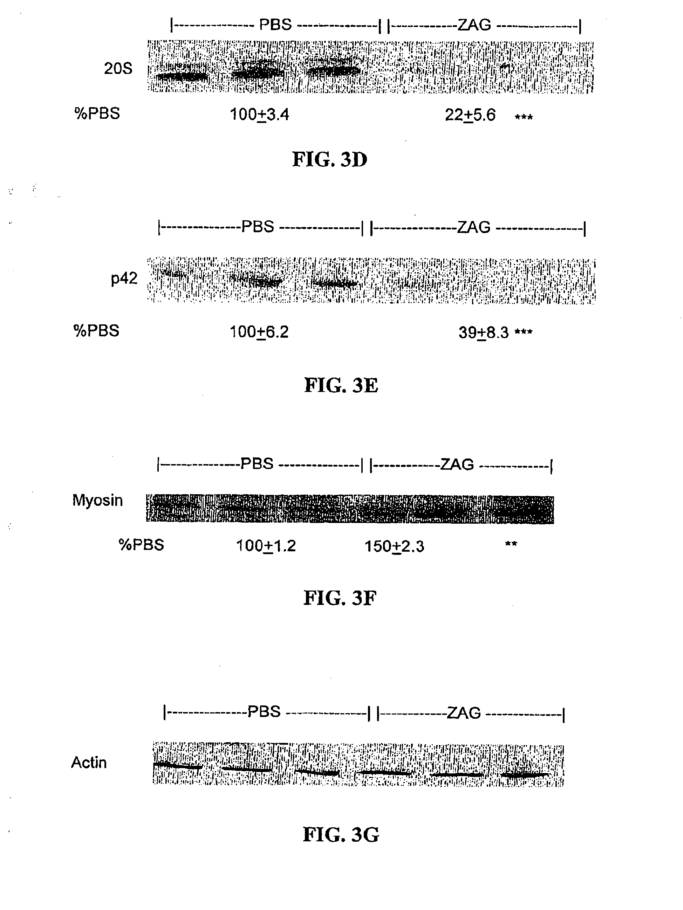

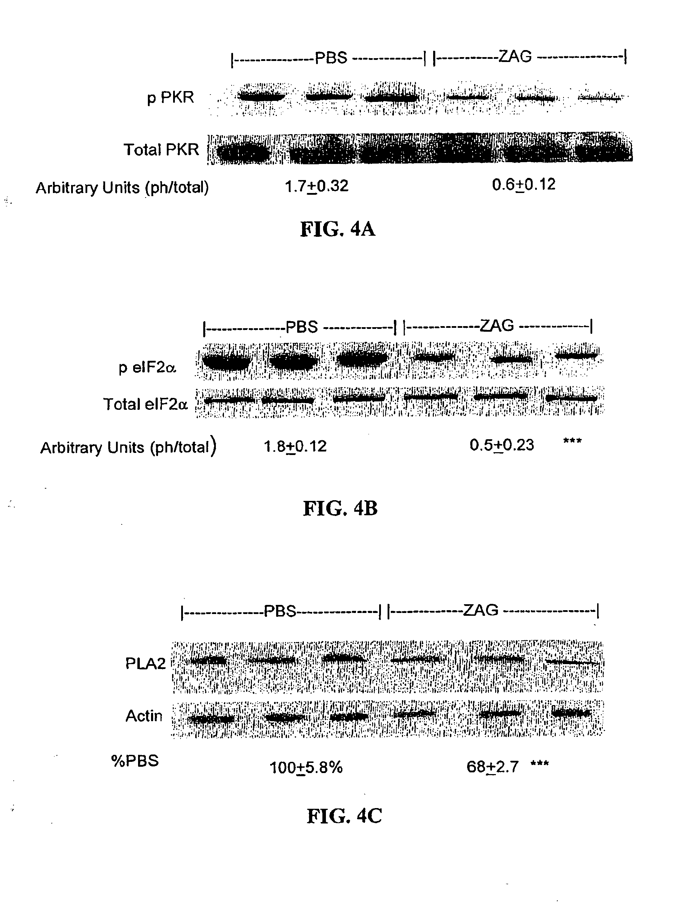

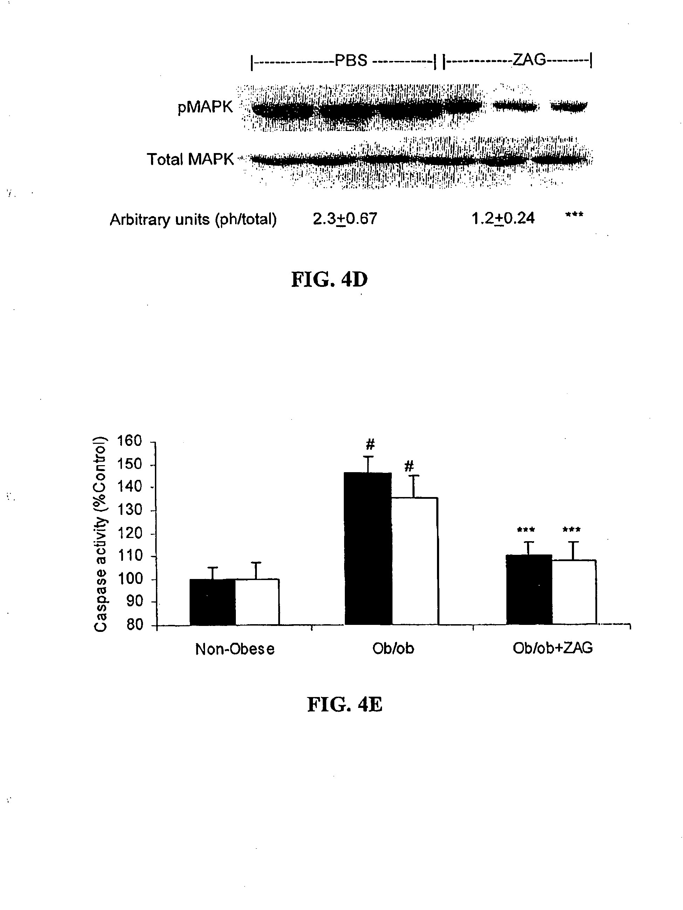

ZAG administration also attenuated the effect of hyperglycemia on skeletal muscle atrophy. Thus ob/ob mice treated with ZAG showed a significant increase in the wet weight of both gastrocnemius and soleus muscles (Table 1). This was associated with over a two-fold increase in protein synthesis in soles muscle (Figure 3A), and a 60% decrease in protein degradation (Figure 3B). Gastrocnemius muscles from mice treated with ZAG showed a decreased activity of the proteasome 'chymotrypsin-like' enzyme activity (Figure 3C), which was not significantly different from that found in non-obese mice, and a decreased expression of both the 20S proteasome α-subunits (Figure 3D), and p42, an ATPase subunit of the 19S regulator (Figure 3E), suggesting a reduced activity of the ubiquitin-proteasome pathway. Myosin levels were increased in ZAG-treated mice (Figure 3F), while actin levels did not change (Figure 3G). In addition there was a reduction in the level of phosphorylated forms of the dsRNA-dependent protein kinase (PKR) (Figure 4A) and eukaryotic initiation factor 2α (eIF2α) (Figure 4B), which have been shown to be responsible for muscle atrophy induced by tumor catabolic factors, and high levels of extracellular glucose. Other enzymes in this pathway including phospholipase A₂ (PLA₂) (Figure 4C), p38 mitogen activated protein kinase (Figure 4D) and caspases-3 and -8 (Figure 4E) were also attenuated in gastrocnemius muscles of ob/ob mice treated with ZAG. These changes were commensurate with a decrease in catabolic signaling in muscle in response to ZAG.

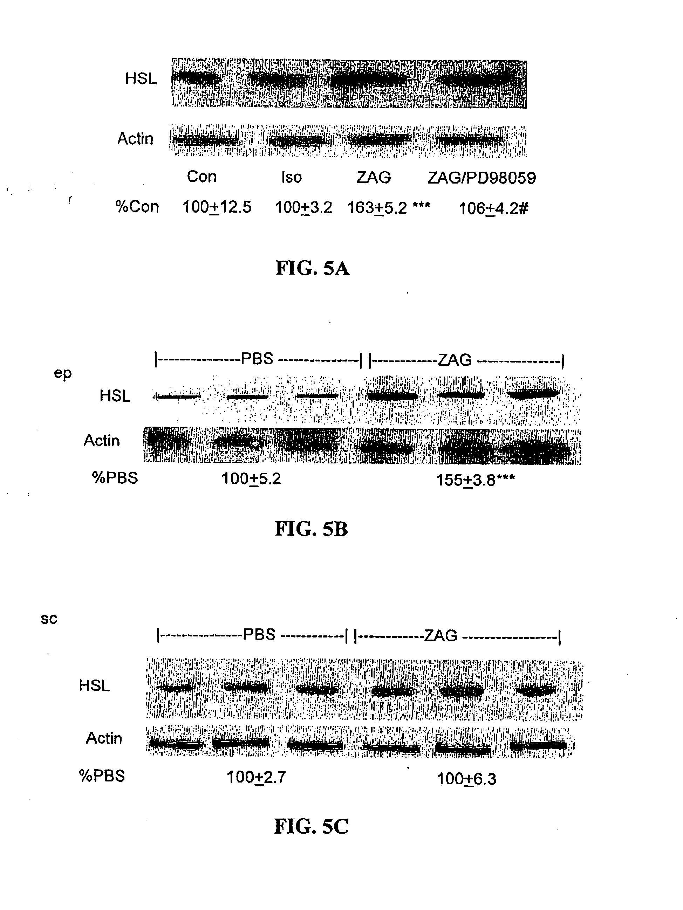

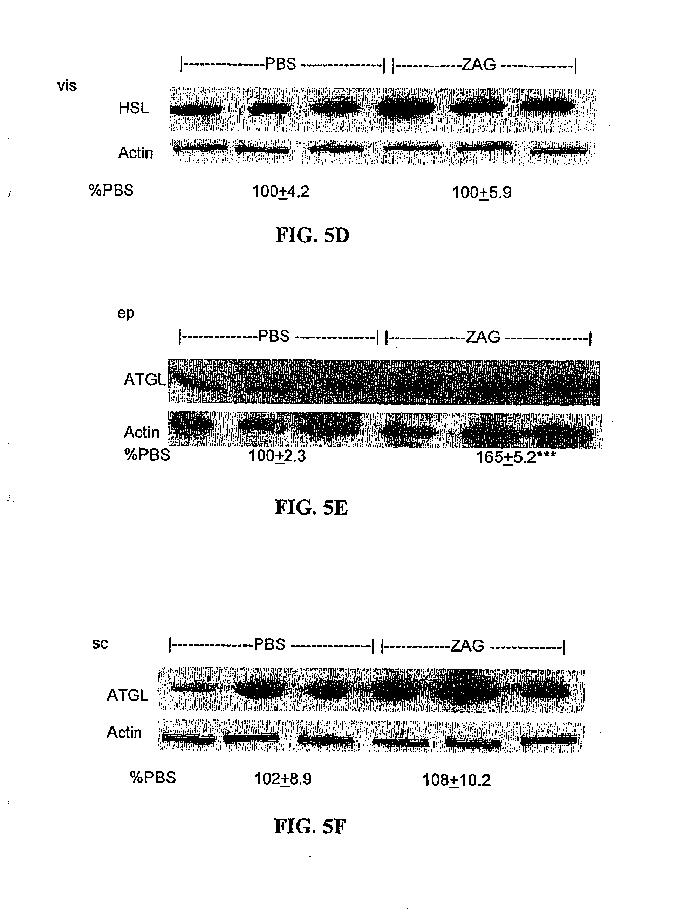

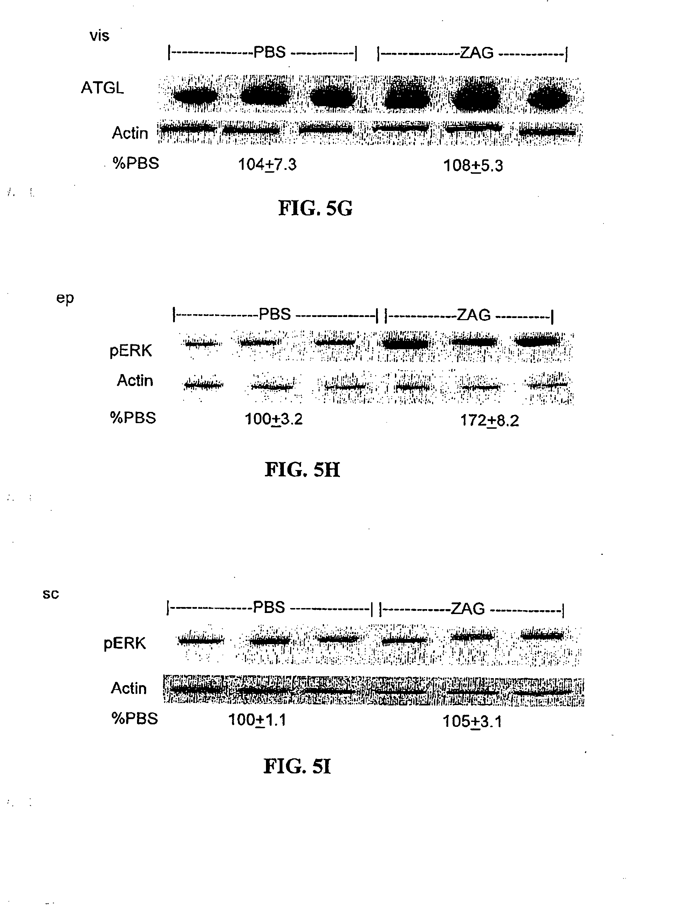

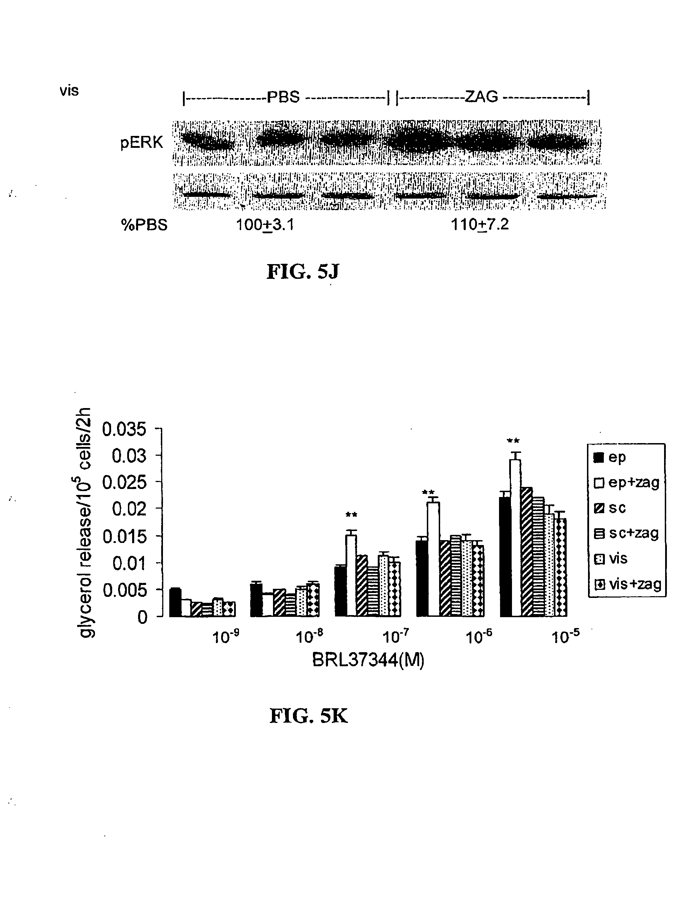

ZAG, but not isoprenaline increased expression of phospho HSL in adipocytes which was completely attenuated by the extracellular signal-regulated kinase (ERK) inhibitor PD98059¹⁴. While ZAG increased expression of HSL in epididymal adipocytes there was no increase in either subcutaneous or visceral adipocytes (Figures 5B-5D). A similar situation was observed with expression of adipose triglyceride lipase (ATGL) (Figures 5E-5G). Expression of HSL and ATGL correlated with expression of the active (phospho) form of ERK (Figures 5H-5J). Expression of HSL and ATGL in epididymal adipocytes correlated with an increased lipolytic response to the β3 agonist, BRL37344 (Figure 5K). This result suggests that ZAG may act synergistically with β3 agonists.

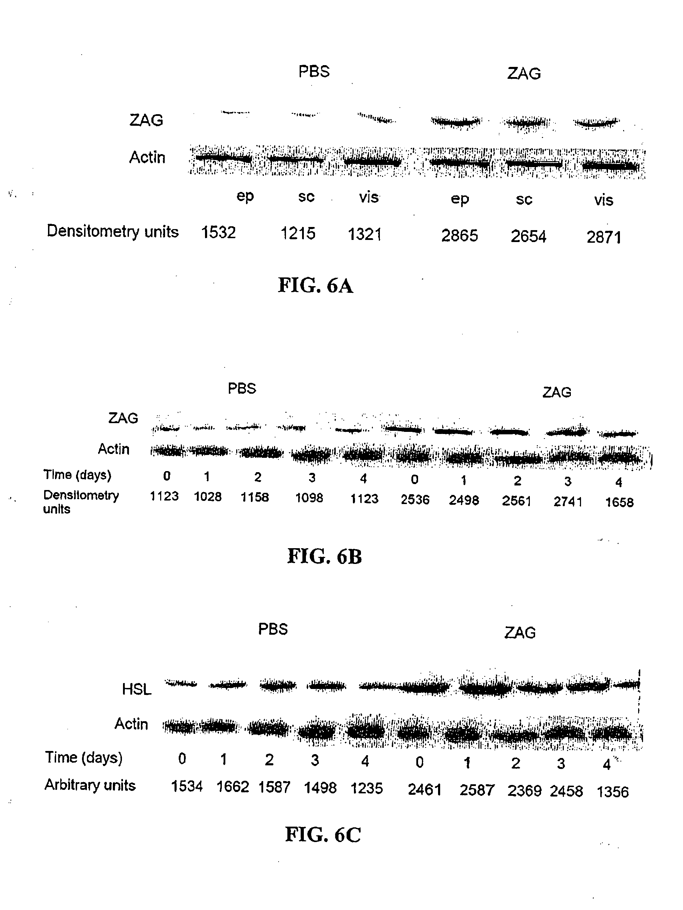

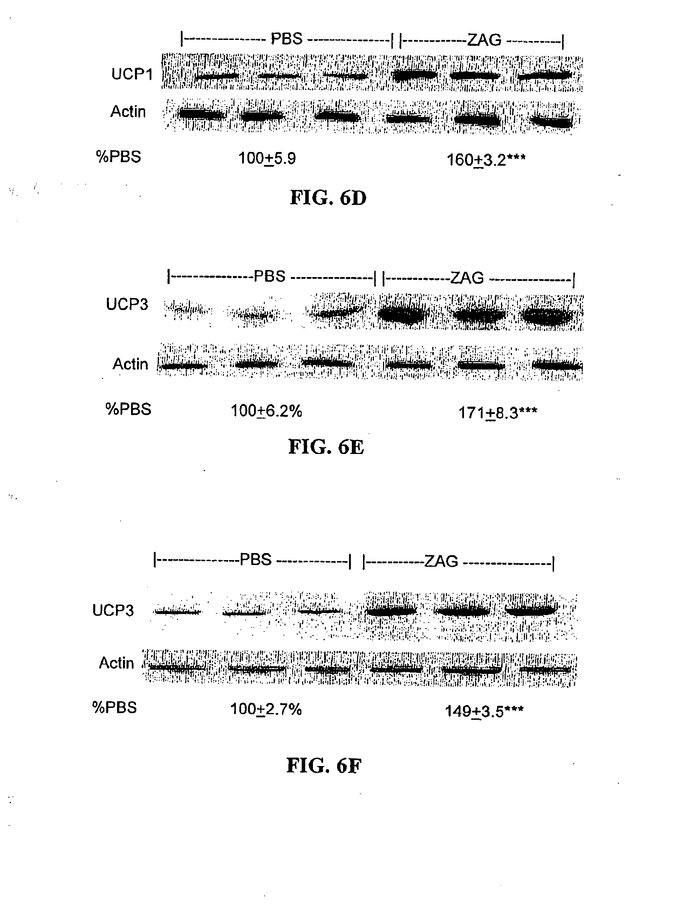

As previously reported, ZAG administration increased its expression in adipose tissue (Figure 6A). ZAG expression remained elevated, for a further 3 days in tissue culture in the absence of ZAG (Figure 6B). Expression of HSL was also elevated in adipocytes for 3 days in tissue culture in the absence of ZAG (Figure 6C). Administration of ZAG increased the expression of UCP1 (Figure 6D) and UCP3 (Figure 6E) in BAT (Figure 6D) and UCP3 in skeletal muscle (Figure 6F). An increased expression of uncoupling proteins would be expected to channel metabolic substrates into heat as observed (Figure 1G).

After 21 days, the plasma metabolite levels in the ob/ob mice were observed (Table 2), with monitored parameters shown in Table 3. A further drop in blood glucose (from 2.03 to 15.2mM) and a rise in glycerol were observed, which seems greater since the control is lower than before. No change in NEFA, TG or insulin was observed at Day 21, as compared to Day 5 (Table 1). It was noted that there is much more insulin in the pancreas in ZAG treated animals showing the drop in plasma insulin, which is not due to lower insulin production (e.g., as would happen with a toxin to pancreatic beta cells), but rather due to the fact that less insulin is needed to control blood glucose in the ZAG treated animals.

**Table 2 - Plasma metabolite and insulin levels in ob/ob mice treated with ZAG at Day 21.**

| | PBS | ZAG | |
|---|---|---|---|
| Glucose (mmol/l) | 24.1 ± 2.3 | 15.2 ± 2.1 | p=0.0085 |
| NEFA(mEq/l) | 0.62 ± 0.008 | 0.22 ± 0.06 | p=0.0025 |
| Glycerol | 290 ± 25.2 | 450 ± 36.2 | p=0.0058 |
| Triglycerides (mmol/l) | 1.72 ± 0.05 | 0.89 ± 0.08 | p=0.0072 |
| Insulin (ng/ml) | 39.5 ± 0.96 | 28.5 ± 0.34 | p=0.0056 |
| Insulin Pancreas (pg/g pancerase) | 6.2 ± 3.2 | 14.5 ± 2.5 | p=0.0035 |

**Table 3 -Parameters monitored in ob/ob mice treated with ZAG at Day 21.**

| Parameter | PBS | ZAG | p |
|---|---|---|---|
| Start weight | 92.5 ± 3.1 | 93.1 ± 1.9 | |
| Finish weight | 89.9 ± 1.3 | 83.95 ± 2.2 | |
| Food (g) | 135 ± 6 | 145 ± 4 | |
| Water (ml) | 268 ± 15 | 259 ± 20 | |
| BAT (g) | 0.36 ± 0.21 | 0.4 ± 0.35 | |
| Gastrocnemius (g) | 0.26 ± 0.15 | 0.39 ± 0.12 | 0.01 |
| Soleus (g) | 0.15 ± 0.06 | 0.18 ± 0.07 | |

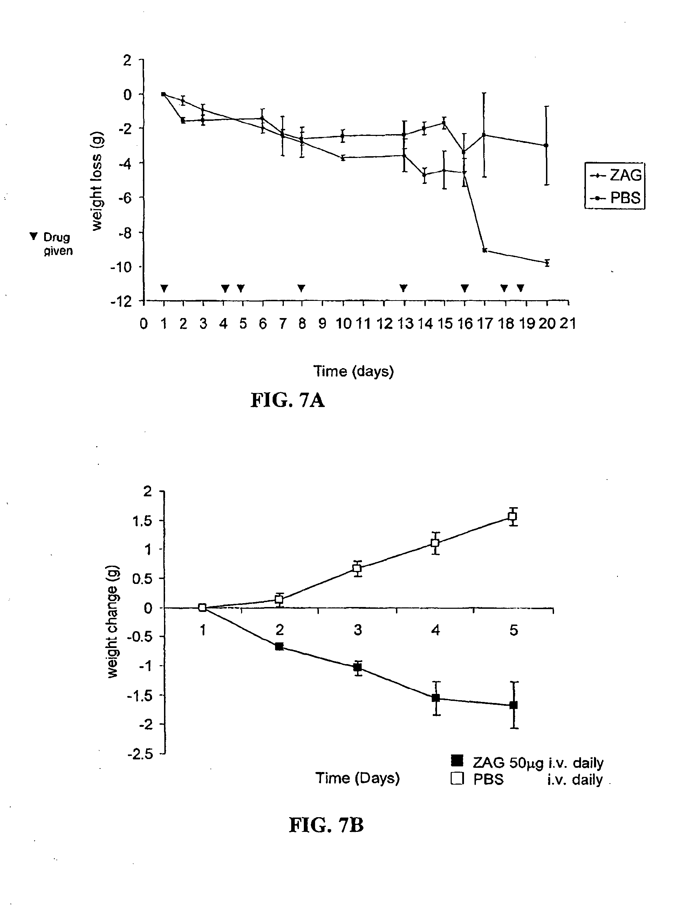

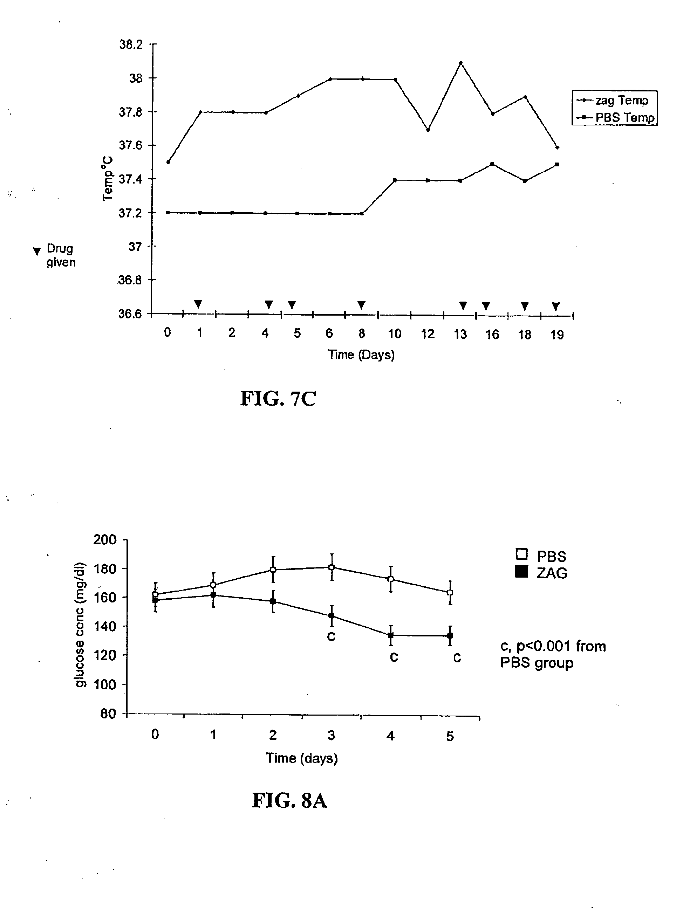

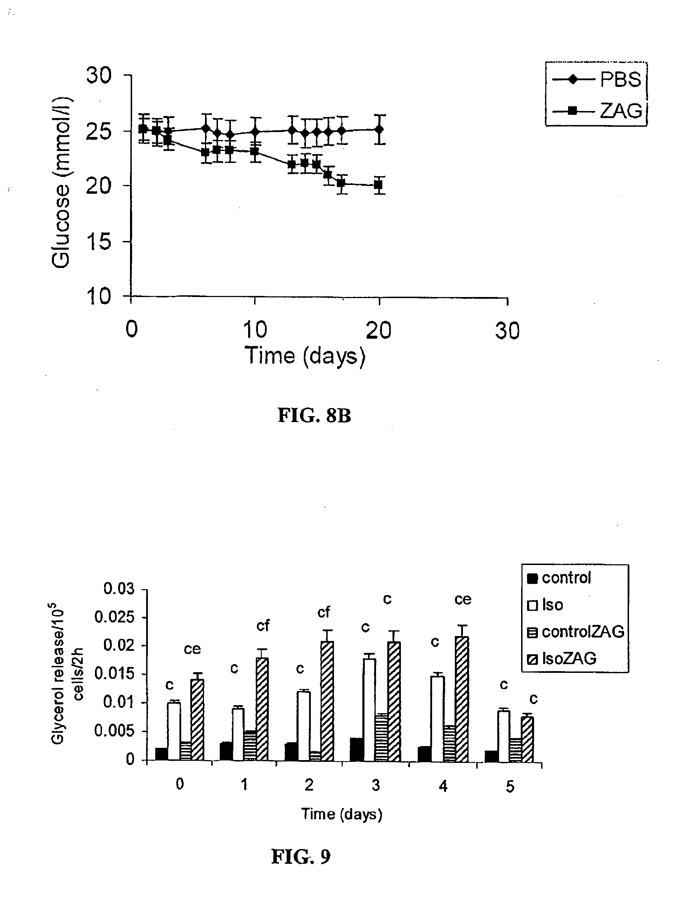

In addition, body temperature of the ob/ob mice increased 0.5° to 1°C (Figure 1G) within four days and peaked at 38.1°C (Figure 7) just before they lost the maximum amount of weight. This would correlate with the weight of brown adipose tissue which increases from 0.33 ± 0.12g in the control to 0.52 ± 0.08g in the ZAG treated animals (Figure 7). The weight of the gastrocnemius muscles was also increased from 0.2 ± 0.05g to 0.7 ± 0.1g, while there was a progressive decrease in urinary glucose excretion (Figures 8A and 8B).

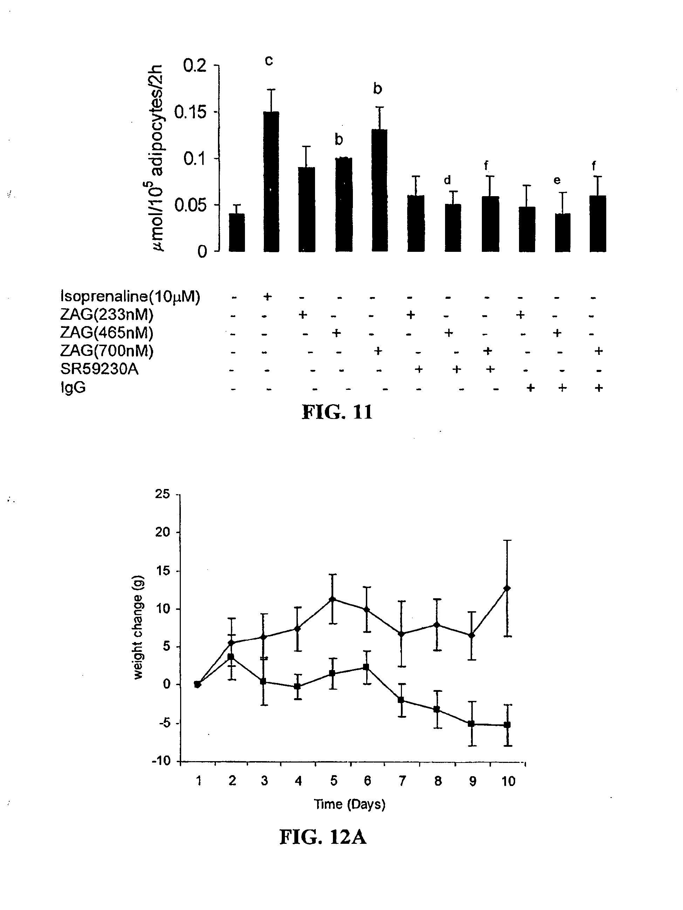

*Results - rats.* The lipolytic effect of human ZAG towards rat epididymal adipocytes in comparison with isoprenaline is shown in Figure 11. At concentrations between 233 and 700nM ZAG produced a dose-related increase in glycerol release, which was attenuated by anti-ZAG monoclonal antibody, showing the specificity of the action. The extent of lipolysis in rat adipocytes was similar to that previously reported in the mouse. As in the mouse, the lipolytic effect of ZAG was completely attenuated by the β3-adrenergic receptor (β3-AR) antagonist SR59230A, suggesting that the action of ZAG was mediated through β3-AR. These results suggest that ZAG may be effective in inducing fat loss in rats.

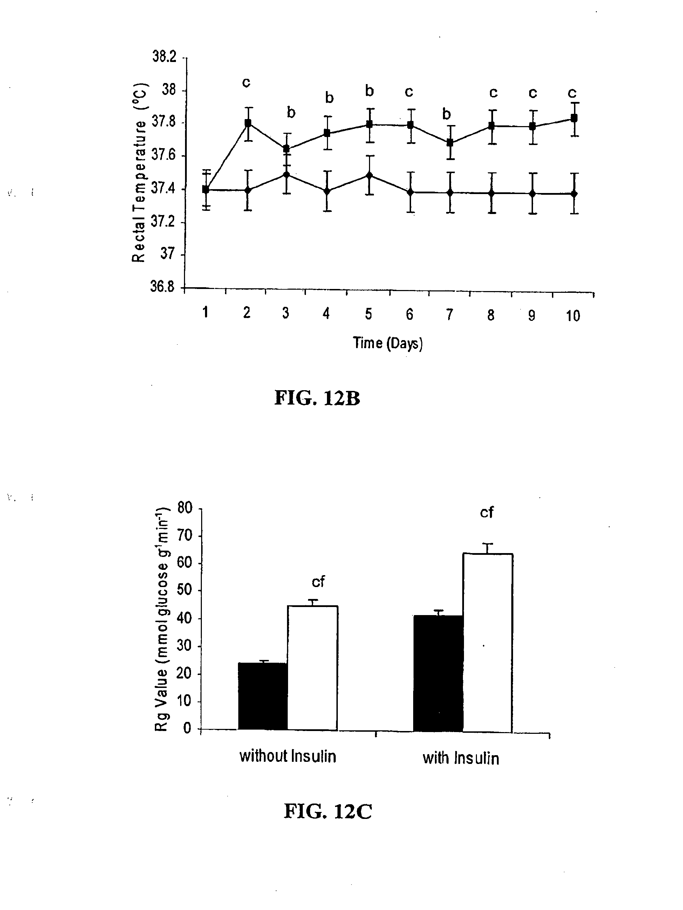

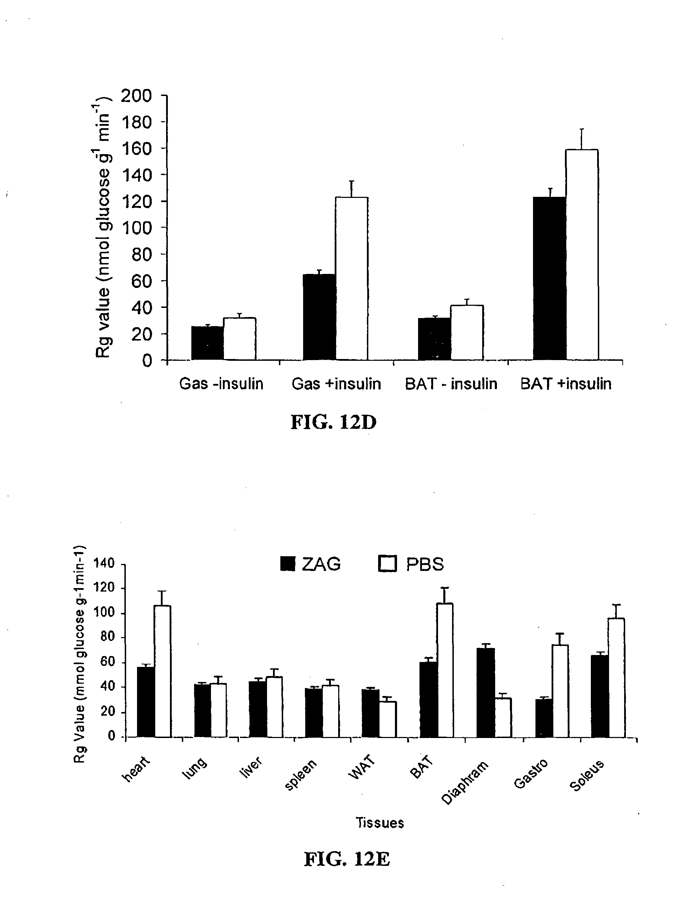

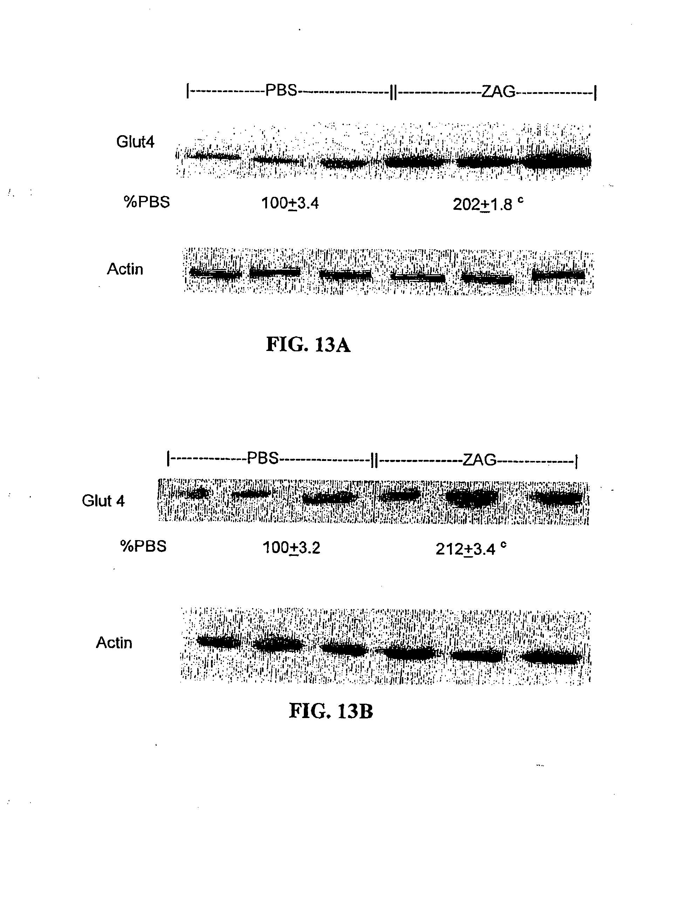

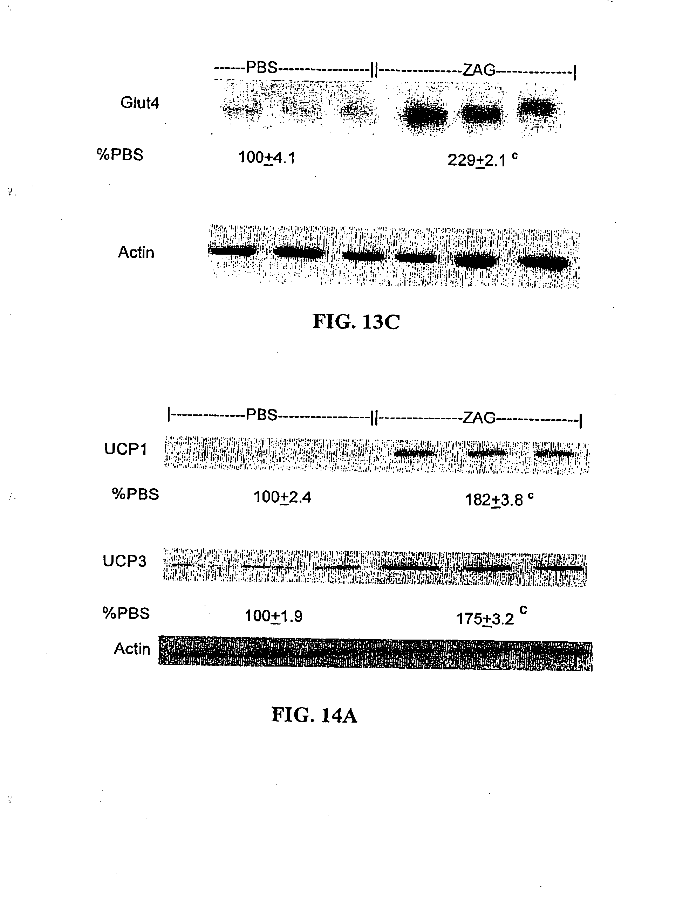

The effect of single daily i.v. injection of ZAG (50µg/100g b.w.) on the body weight of mature male Wistar rats (540±83g) is shown in Figure 12A. Compared with control rats administered the same volume of solvent (PBS), rats administered ZAG showed a progressive decrease in body weight, such that after 10 days, while rats treated with PBS showed a 13g increase in body weight, animals treated with ZAG showed a 5g decrease in body weight (Table 4). There was no difference in food (ZAG: 102±32g; PBS:98±25g) or water (ZAG: 135±35ml; PBS: 125±25ml) intake between the two groups during the course of the study, but ZAG-treated animals showed a consistent 0.4°C elevation in body temperature, which was significant within 24h of the first administration of ZAG (Figure 12B), indicating an elevated energy expenditure. Body composition analysis (Table 4) showed that the loss of body weight induced by ZAG was due to a loss of carcass fat, which was partially offset by a significant increase in lean body mass. There was a 50% increase in plasma glycerol concentration in rats treated with ZAG (Table 5), indicative of an increased lipolysis, but a 55% decrease in plasma levels of non-esterified fatty acids (NEFA), suggesting an increased utilisation. Plasma levels of glucose and triglycerides were also reduced by 36-37% (Table 5), also suggesting an increased utilization. There was a significant increase in the uptake of 2-deoxygluocse into epididymal adipocytes of rats treated with ZAG for 10 days, which was increased in the presence of insulin (Figure 12C). However, there was no significant difference in glucose uptake into adipocytes from ZAG or PBS treated animals in the presence of insulin (Figure 12C). There was a small, non-significant increase in glucose uptake into gastrocnemius muscle and BAT of rats treated with ZAG in comparison with PBS controls, but a significant increase in uptake in the presence of insulin (Figure 12D). These results suggest that the decrease in blood glucose is due to increased utilization by BAT, WAT and skeletal muscle, and this is supported by an increased expression of glucose transporter 4 (GLUT4) in all three tissues (Figure 13).

**Table 4 - Body composition of male rats after treatment with either PBS or ZAG**

| Treatment | Starting weight (g) | Final weight (g) | Weight change (g) | Water | | Fat | | Non fat | |
|---|---|---|---|---|---|---|---|---|---|
| | | | | (g) | (%) | (g) | (%) | (g) | (%) |
| PBS | 510±30 | 523±2 | +13±3 | 326±32 | 62±2 | 105±14 | 20±3 | 90±6 | 17±3 |
| ZAG | 530±45 | 525±1 | -5±1 | 331±5 | 63±3 | 92±5^{b} | 18±1 | 96±2^{a} | 18±2 |

| | | | | | | | | | |
|---|---|---|---|---|---|---|---|---|---|
| Differences from animals treated with PBS are shown as a, p<0.05 or b, p<0.01 | | | | | | | | | |

**Table 5 - Plasma metabolite and insulin levels in rats treated with either PBS or ZAG for 10 days**

| Metabolite | PBS | ZAG |
|---|---|---|
| Glucose (mmol/l) | 25.5±2.3 | 16.2±2.1^{c} |
| Trigylcerides (mmol/l) | 1.75±0.01 | 1.1±0.09^{a} |
| Glycerol (umol/l) | 300±52 | 450±51^{c} |
| NEFA (mEq/l) | 0.58±0.008 | 0.26±0.06^{b} |

| | | |
|---|---|---|
| Differences from animals treated with PBS are shown as either a, p<0.05; b, p<0.01 or c, p<0.001 | | |

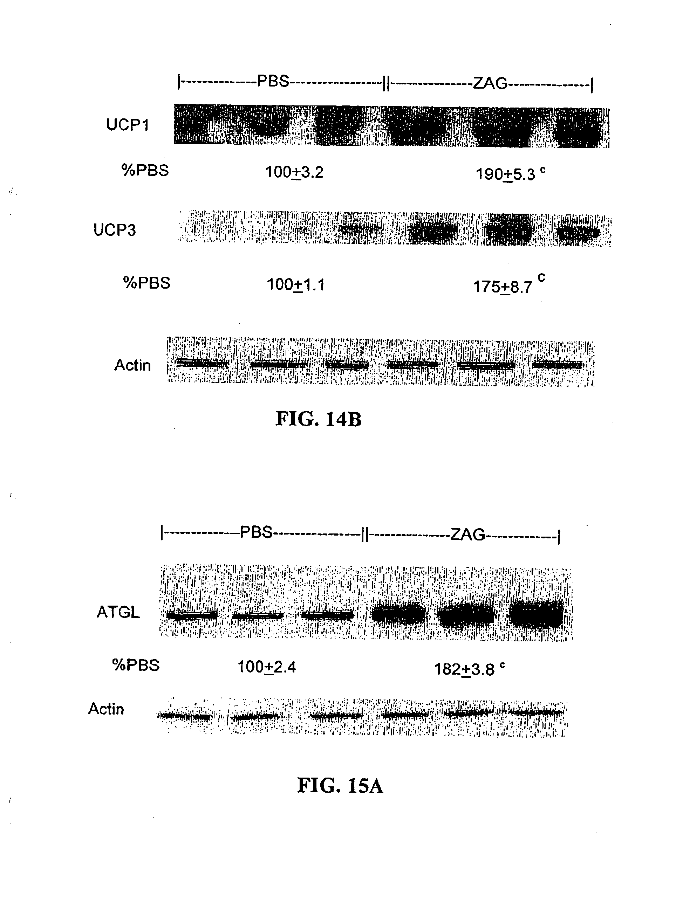

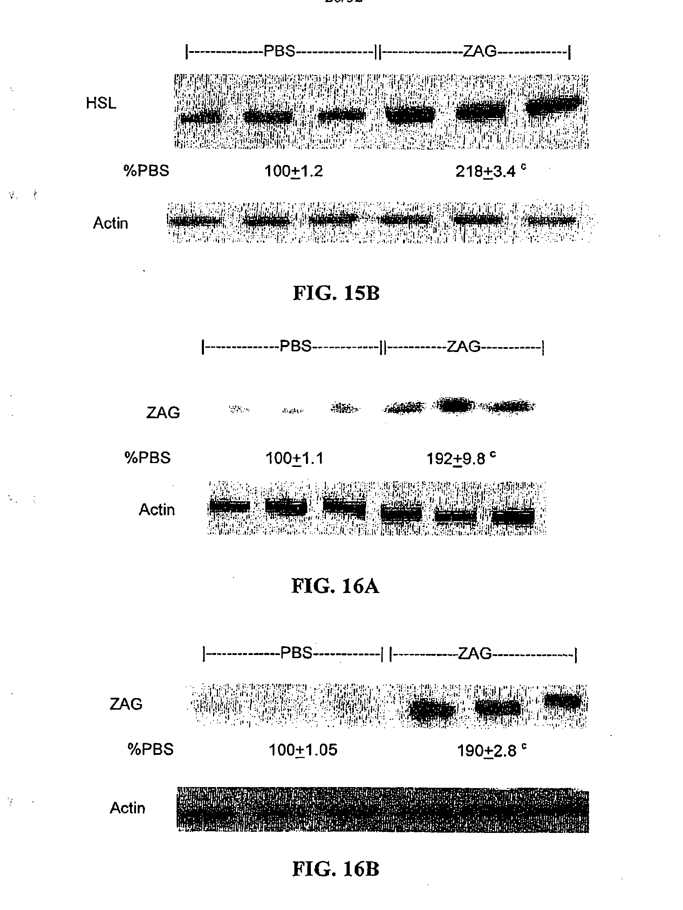

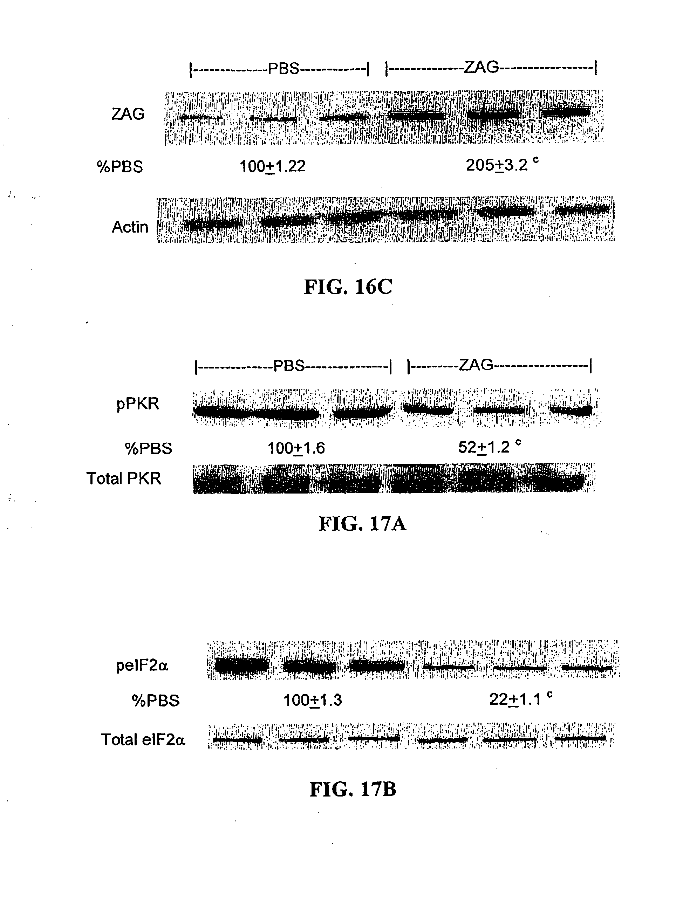

ZAG administration increased expression of the uncoupling proteins (UCP)-1 and - 3 in both BAT and WAT by almost two-fold (Figures 13A and 13B), which would contribute to increased substrate utilization. In rats treated with ZAG there was also an increased expression of the lipolytic enzymes adipose triglyceride lipase (ATGL) and hormone sensitive lipase (HSL) in epididymal adipose tissue (Figure 15), again with a two-fold increase. ATGL is mainly responsible for the hydrolysis of the first ester bond in a triacylglycerol molecule forming diacylgylcerol, while its conversion to monacylglycerol is carried out by HSL. Expression of ZAG was also significantly increased in skeletal muscle, (Figure 16A), WAT (Figure 16B) and BAT (Figure 16C) of rats treated with ZAG for 10 days, showing that exogenous ZAG boosts its own production in peripheral tissues.

There was a significant reduction in the expression of the phosphorylated forms of both dsRNA-dependent protein kinase (PKR) and eukaryotic initiation factor 2 (eIF2) on the α-subunit in gastrocnemius muscle of rats administered ZAG, while the total amount did not change (Figures 17A and 17B). Similar changes have been observed in ob/ob mice administered ZAG (unpublished results) and were consistent with a depression of protein degradation and increase in protein synthesis in skeletal muscle.

### EXAMPLE 2

### Interval Administration of Zinc-α₂-glycoprotein

It was observed that long-term daily administration of ZAG in ob/ob mice results in a cessation of weight loss. As such, it was determined that a break of 3-4 days followed by re-infusion ZAG resulted in continued weight loss and amelioration of the symptoms associated with hyperglycemia.

While not wanting to be limited by theory, it may be that the subjects are receiving too much ZAG or that there is receptor desensitization as is seen with TNF. A pilot study was performed with 2 mice in each group to determine optimal scheduling of ZAG delivery. An 8-10g weight loss from a 90g mouse was observed in about 3 weeks.

Adipocytes were removed from mice after 5 days of ZAG and their responsiveness to isoprenaline (iso) was measured after culture in the absence of ZAG (Figure 9). The responsiveness to iso is higher in ZAG treated mice and this continues for a further 4 days (which was when expression of ZAG and HSL were increased) and then falls on day 5 (when expression was not increased) down to values of PBS control.

### EXAMPLE 3

### Zinc-α₂-glycoprotein attenuates muscle atrophy in ob/ob mouse

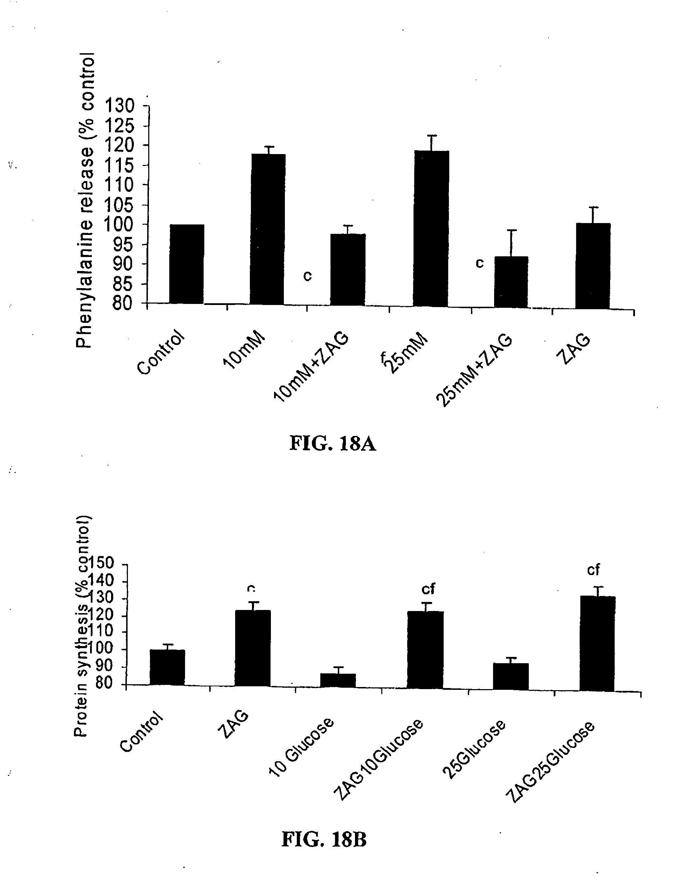

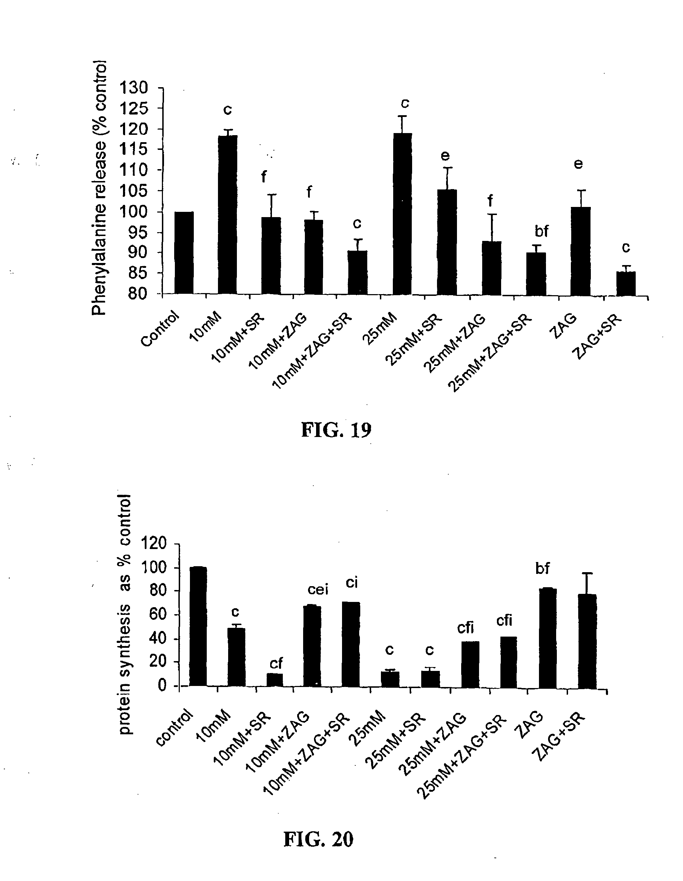

This example demonstrates the mechanism by which ZAG attenuates muscle atrophy in the ob/ob mouse using a newly *developed in vitro* model (Russell et al, Exp. Cell Res. 315, 16-25, 2009). This utilizes murine myotubes subjected to high concentrations of glucose (10 or 25mM). As shown in Figure 18 high glucose stimulates an increase in protein degradation (Figure 18A), and depresses protein synthesis (Figure 18B), and both of these effects were completely attenuated by ZAG (25µg/ml). It was therefore determined if the effect of ZAG was mediated through a β3-AR using the antagonist SR59230A. However the SR compound (*i.e.,* SR59230A) can also act as a β-agonist, which it seemed to do in these experiments. Thus protein degradation induced by both 10 and 25mM glucose was attenuated by both ZAG and the SR compound, and the combination was additive rather than antagonistic (Figure 19). For protein synthesis (Figure 20) the SR compound seems to be similar to ZAG with no evidence of reversal, while with 10mM glucose the SR compound causes an increase in the depression of protein synthesis.

### EXAMPLE 4

### Zinc-α₂-glycoprotein attenuates ROS formation

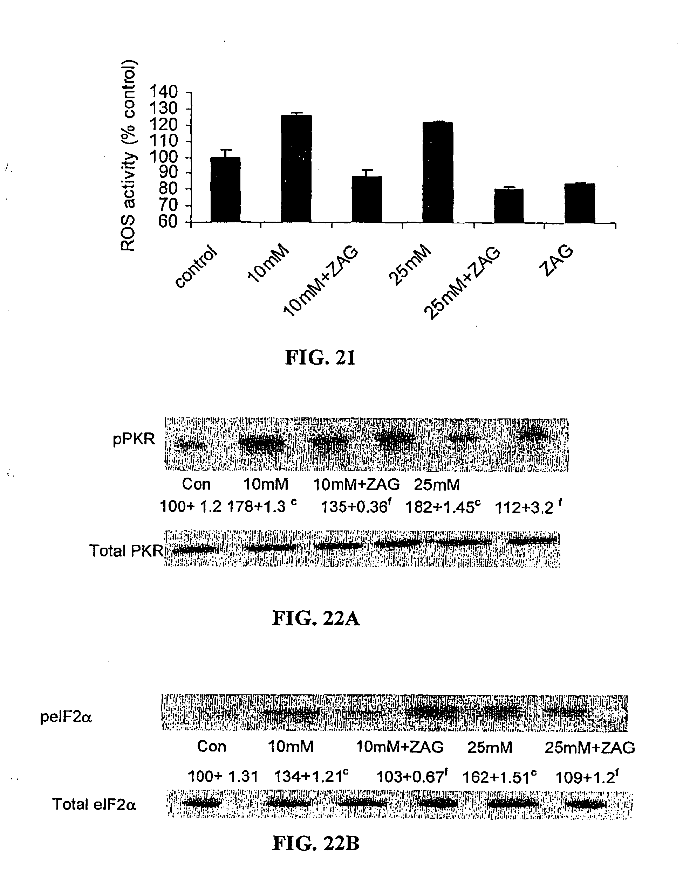

It has been shown that formation of reactive oxygen species (ROS) is important in protein degradation induced by high glucose load. The data in Figure 21 shows that ZAG completely attenuates the increase in ROS produced by glucose, corresponding with the decrease in protein degradation (Figure 18A). High glucose also induces activation (phosphorylation) of PKR (Figure 22A) and the subsequent phosphorylation of eIF2α (Figure 22B) as is seen in skeletal muscle of ob/ob mice, which was also attenuated by ZAG. These results suggest that this *in vitro* model will be useful to study how ZAG affects muscle mass at the molecular level.

### EXAMPLE 5

### Zinc-α₂-glycoprotein increases insulin tolerance

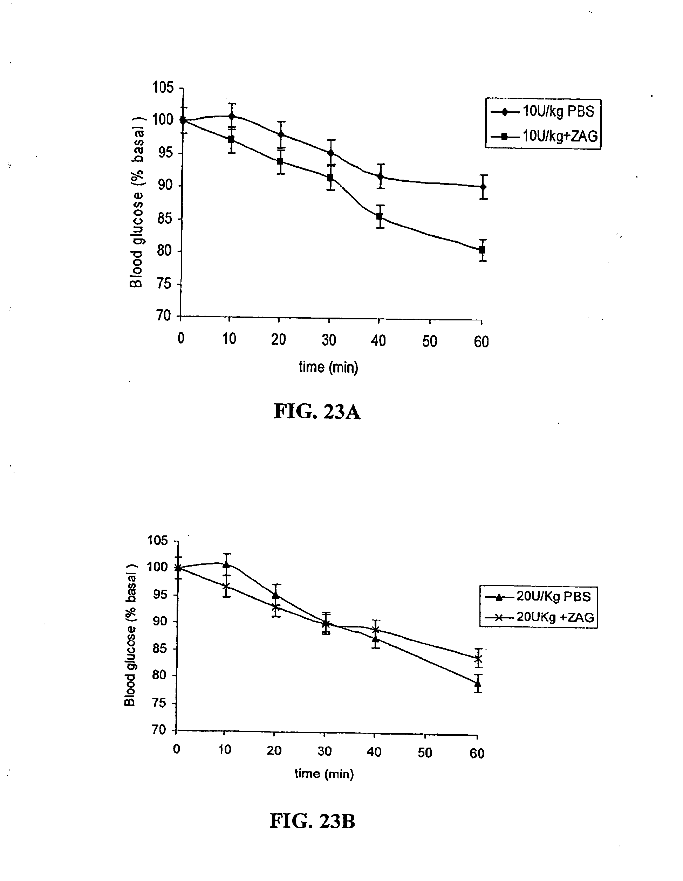

An insulin tolerance test was also carried out in ob/ob mice administered ZAG for 3 days (Figure 23). Animals were administered two doses of insulin (10 and 20 U/kg) by i.p. injection and blood glucose was measured over the next 60 min. As can be seen (Figure 23A) animals treated with ZAG showed an increased sensitivity to insulin (10 U/kg) than those given PBS. At the higher concentration of insulin (20U/kg) this difference disappeared (Figure 23B). The glucose disappearance curve for 20 U/kg + PBS was almost identical to 10 U/kg + ZAG, so at this dose level ZAG is reducing the requirement for insulin by 50%, but this can be overcome by giving more insulin.

### EXAMPLE 6

### 5-day administration of zinc-α₂-glycoprotein

The data shown in Attachment 1 is from a 5 day study where ZAG was administered at 35µg per day i.v. on a daily basis for 5 days. At the end of the experiment tissues were removed and blotted, or functional assays were carried out with isolated adipocytes. As can be seen in Figure 6A, ZAG administration increased its expression in epididymal (ep), subcutaneous (sc) and visceral (vis) fat about two-fold. When ep adipocytes were prepared and maintained in tissue culture (RPMI 1640 +10% FCS) ZAG expression was maintained for a further 3 days, even though no ZAG was added to the culture medium (Figure 6B). In addition adipocytes from ZAG treated mice showed an increased response to isoprenaline (10µM), and this was also maintained for 4 days in tissue culture in the absence of ZAG (Figure 9). The increased response to isoprenaline is due to an increased expression of HSL by ZAG, and this was also maintained in tissue culture for 4 days in the absence of ZAG (Figure 6C). These results show that the effects of ZAG are maintained for a further 3 days when ZAG is withdrawn and therefore it need not be administered on a daily basis. In fact, as discussed above, too much ZAG is more likely to lead to resistance rather than an increased response.

An increased expression of HSL was only seen in ep adipocytes after 5 days ZAG (Figures 5B-5D), as was ATGL (Figures 5E-5G). There was an increase in expression of pERK only in ep adipose tissue (Figures 5H-5J), and an inhibitor of pERK (PD98059 10µM) attenuated the increase in expression of HSL in ep adipocytes incubated with ZAG for 3h (Figure 5A). ZAG increased expression of UCP1 and UCP3 in BAT (Figures 6D and 6E) and muscle (Figure 6F) which would account for the increase in body temperature and fall in TG and NEFA in serum despite the increase in lipolysis.

Although the invention has been described with reference to the above examples, it will be understood that modifications and variations are encompassed within the spirit and scope of the invention. Accordingly, the invention is limited only by the following claims.

## Claims

1. A polypeptide having the sequence as shown in SEQ ID NO: 1 for use in ameliorating symptoms of hyperglycemia by administering to the subject a therapeutically effective dosage for a period of 21 days or longer, wherein there is a continuous amelioration of symptoms of hyperglycemia over the course of treatment.

2. The polypeptide for use as claimed in claim 1, wherein the dosage is daily for 21 days, or every other day, every 2 days, or every 3 days for 21 days.

3. The polypeptide for use as claimed in of claim 1, wherein the amelioration of symptoms comprises one or more of symptoms selected from the group consisting of a decrease in serum levels of glucose, a decrease in serum levels of triglycerides, a decrease in serum levels of insulin, and a decrease in serum levels of non-esterified fatty acids, as compared to serum levels prior to treatment.

4. The polypeptide for use as claimed in claim 1, wherein the amelioration of symptoms comprises an increase in body temperature of 0.4° to 1°C within 4 day of initiating treatment, as compared to body temperature prior to treatment.

5. The polypeptide for use as claimed in claim 1, wherein the amelioration of symptoms comprises a decrease in plasma insulin levels within 3 days of initiating treatment, as compared to plasma insulin levels prior to treatment.

6. The polypeptide for use as claimed in claim 5, wherein there is a 36% decrease in plasma insulin levels as compared to plasma insulin levels prior to treatment.

7. The polypeptide for use as claimed in claim 5, wherein there is a 36% decrease in plasma glucose and triglyceride levels as compared to plasma glucose and triglyceride levels prior to treatment.

8. The polypeptide for use as claimed in claim 1, wherein the amelioration of symptoms comprises normalization of blood glucose levels and insulin secretion in response to intravenous glucose within 3 days of initiating treatment.

9. The polypeptide for use as claimed in claim 1, wherein the amelioration of symptoms comprises an increase in pancreatic insulin levels, as compared to pancreatic insulin levels prior to treatment.

10. The polypeptide for use as claimed in claim 1, wherein the amelioration of symptoms comprises increased expression of uncoupling protein-1 (UCP1) and uncoupling protein-3 (UCP3) in brown adipose tissue, as compared to expression of UCP1 and UCP3 prior to treatment.

11. The polypeptide for use as claimed in claim 1, wherein the amelioration of symptoms comprises increased expression of UCP3 in skeletal muscle, as compared to expression of UCP3 prior to treatment.

12. The polypeptide for use as claimed in claim 1, wherein the polypeptide is administered twice daily.

13. The polypeptide for use as claimed in claim 1, wherein the polypeptide is administered intravenously, subcutaneously, intraperitoneally, or orally.

14. The polypeptide for use as claimed in claim 1, wherein the polypeptide is administered once every three days.

15. The polypeptide for use as claimed in claim 1, wherein the polypeptide is glycosylated.

## Patentansprüche

1. Polypeptid mit der wie in SEQ ID NR: 1 dargestellten Sequenz zur Verwendung bei der Besserung von Symptomen der Hyperglykämie durch die Verabreichung einer therapeutisch wirksamen Dosis an ein Subjekt über einen Zeitraum von 21 Tagen oder länger, wobei im Verlauf der Behandlung eine kontinuierliche Besserung der Symptome der Hyperglykämie zu beobachten ist.

2. Polypeptid zur Verwendung nach Anspruch 1, wobei die Dosierung täglich für 21 Tage, oder jeden anderen Tag, alle 2 Tage oder alle 3 Tage für 21 Tage entspricht.

3. Polypeptid zur Verwendung nach Anspruch 1, wobei die Besserung der Symptome eines oder mehrere Symptome umfasst, die ausgewählt sind aus der Gruppe bestehend aus einer Reduktion des Glukosespiegels im Serum, einer Reduktion des Triglyceridspiegels im Serum, einer Reduktion des Insulinspiegels im Serum und einer Reduktion des Spiegels an nicht veresterten Fettsäuren im Serum im Vergleich zu den jeweiligen Serumspiegeln vor Beginn der Behandlung.

4. Polypeptid zur Verwendung nach Anspruch 1, wobei die Besserung der Symptome einen Anstieg der Körpertemperatur um 0,4°C bis 1°C innerhalb von 4 Tagen nach dem Beginn der Behandlung im Vergleich zu der Körpertemperatur vor Beginn der Behandlung umfasst.

5. Polypeptid zur Verwendung nach Anspruch 1, wobei die Besserung der Symptome eine Reduktion des Insulinspiegels im Plasma innerhalb von 3 Tagen nach Beginn der Behandlung im Vergleich zu dem Insulinspiegel im Plasma vor Beginn der Behandlung umfasst.

6. Polypeptid zur Verwendung nach Anspruch 5, wobei der Insulinspiegel im Plasma im Vergleich zu dem Insulinspiegel im Plasma vor Beginn der Behandlung um 36% reduziert ist.

7. Polypeptid zur Verwendung nach Anspruch 5, wobei die Glukose-und Triglyceridspiegel im Plasma im Vergleich zu den Glukose- und Triglyceridspiegeln im Plasma vor Beginn der Behandlung um 36% reduziert ist.

8. Polypeptid zur Verwendung nach Anspruch 1, wobei die Besserung der Symptome die Normalisierung des Glukosespiegels im Blut sowie der Insulinsekretion in Reaktion auf intravenös verabreichte Glukose innerhalb von 3 Tagen nach Beginn der Behandlung umfasst.

9. Polypeptid zur Verwendung nach Anspruch 1, wobei die Besserung der Symptome einen Anstieg des Insulinspiegels im Pankreas im Vergleich zu dem Insulinspiegel im Pankreas vor Beginn der Behandlung umfasst.

10. Polypeptid zur Verwendung nach Anspruch 1, wobei die Besserung der Symptome eine erhöhte Expression von UCP1 *(uncoupling protein-*1) und UCP3 *(uncoupling protein-3*) im braunen adipösen Gewebe im Vergleich zu der Expression von UCP1 und UCP3 im braunen adipösen Gewebe vor Beginn der Behandlung umfasst.

11. Polypeptid zur Verwendung nach Anspruch 1, wobei die Besserung der Symptome eine erhöhte Expression von UCP3 in der Skelettmuskulatur im Vergleich zu der Expression von UPC3 in der Skelettmusku-latur vor dem Beginn der Behandlung umfasst.

12. Polypeptid zur Verwendung nach Anspruch 1, wobei das Polypeptid zwei Mal pro Tag verabreicht wird.

13. Polypeptid zur Verwendung nach Anspruch 1, wobei das Polypeptid intravenös, subkutan, intraperitoneal oder oral verabreicht wird.

14. Polypeptid zur Verwendung nach Anspruch 1, wobei das Polypeptid ein Mal alle drei Tage verabreicht wird.

15. Polypeptid zur Verwendung nach Anspruch 1, wobei das Polypeptid glykosyliert ist.

## Revendications

1. Polypeptide possédant la séquence représentée par SEQ ID NO : 1 pour une utilisation dans l'amélioration des symptômes de l'hyperglycémie par l'administration au sujet d'une dose thérapeutiquement efficace pendant une période de 21 jours ou plus, dans lequel il y a une amélioration continue des symptômes de l'hyperglycémie au cours du traitement.

2. Polypeptide pour une utilisation selon la revendication 1, dans lequel la dose est quotidienne pendant 21 jours, un jour sur deux, tous les 2 jours ou tous les 3 jours pendant 21 jours.

3. Polypeptide pour une utilisation selon la revendication 1, dans lequel l'amélioration des symptômes comprend un ou plusieurs des symptômes choisis dans le groupe constitué d'une diminution des taux sériques de glucose, d'une diminution des taux sériques de triglycérides, d'une diminution des taux sériques d'insuline et d'une diminution des taux sériques d'acides gras non estérifiés, par rapport aux taux sériques avant le traitement.

4. Polypeptide pour une utilisation selon la revendication 1, dans lequel l'amélioration des symptômes comprend l'augmentation de la température corporelle de 0,4°C à 1°C en moins de 4 jours après le début du traitement, par rapport à la température corporelle avant le traitement.

5. Polypeptide pour une utilisation selon la revendication 1, dans lequel l'amélioration des symptômes comprend une diminution des taux plasmatiques d'insuline en moins de 3 jours après le début du traitement, par rapport aux taux plasmatiques d'insuline avant le traitement.

6. Polypeptide pour une utilisation selon la revendication 5, dans lequel il y a une diminution de 36% des taux plasmatiques d'insuline par rapport aux taux plasmatiques d'insuline avant le traitement.

7. Polypeptide pour une utilisation selon la revendication 5, dans lequel il y a une diminution de 36% des taux plasmatiques de glucose et de triglycérides par rapport aux taux plasmatiques de glucose et de triglycérides avant le traitement.

8. Polypeptide pour une utilisation selon la revendication 1, dans lequel l'amélioration des symptômes comprend la normalisation de la glycémie et de la sécrétion d'insuline en réponse à du glucose intraveineux en moins de 3 jours après le début du traitement.

9. Polypeptide pour une utilisation selon la revendication 1, dans lequel l'amélioration des symptômes comprend une augmentation des taux d'insuline pancréatique par rapport aux taux d'insuline pancréatique avant le traitement.

10. Polypeptide pour une utilisation selon la revendication 1, dans lequel l'amélioration des symptômes comprend une augmentation de l'expression de la protéine découplante 1 (UCP1) et de la protéine découplante 3 (UCP3) dans le tissu adipeux brun, par rapport à l'expression de l'UCP1 et de l'UCP3 avant le traitement.

11. Polypeptide pour une utilisation selon la revendication 1, dans lequel l'amélioration des symptômes comprend une augmentation de l'expression de l'UCP3 dans le muscle squelettique, par rapport à l'expression de l'UCP3 avant le traitement.

12. Polypeptide pour une utilisation selon la revendication 1, dans lequel le polypeptide est administré deux fois par jour.

13. Polypeptide pour une utilisation selon la revendication 1, dans lequel le polypeptide est administré par voie intraveineuse, sous-cutanée, intrapéritonéale ou orale.

14. Polypeptide pour une utilisation selon la revendication 1, dans lequel le polypeptide est administré une fois tous les trois jours.

15. Polypeptide pour une utilisation selon la revendication 1, dans lequel le polypeptide est glycosylé.


### SEQUENCE LISTING

<110> ASTON UNIVERSITY TISDALE, Michael j. RUSSELL, Steve
<120> GLYCOPROTEINS HAVING LIPID MOBILIZING PROPERTIES AND THERAPEUTIC USES THEREOF
<130> HALSA1120-2WO
<150> US 61/253,023
   <151> 2009-10-19
<150> US 61/112,623
   <151> 2008-11-07
<160> 6
<170> PatentIn version 3.5
<210> 1
   <211> 276
   <212> PRT
   <213> Homo sapiens
<400> 1
   Gln Glu Asn Gln Asp Gly Arg Tyr Ser Leu Thr Tyr Ile Tyr Thr Gly
   1 5 10 15
   Leu Ser Lys His Val Glu Asp Val Pro Ala Phe Gln Ala Leu Gly Ser
   20 25 30
   Leu Asn Asp Leu Gln Phe Phe Arg Tyr Asn Ser Lys Asp Arg Lys Ser
   35 40 45
   Gln Pro Met Gly Leu Trp Arg Gln Val Glu Gly Met Glu Asp Trp Lys
   50 55 60
   Glu Asp Ser Gln Leu Gln Lys Ala Arg Glu Asp Met Glu Thr Leu Lys
   65 70 75 80
   Asp Ile Val Glu Tyr Tyr Asn Asp Ser Asn Gly Ser His Val Leu Gln
   85 90 95
   Gly Arg Phe Gly Cys Glu Ile Glu Asn Asn Arg Ser Ser Gly Ala Phe
   100 105 110
   Trp Lys Tyr Tyr Tyr Asp Gly Lys Asp Tyr Ile Glu Phe Asn Lys Glu
   115 120 125
   Ile Pro Ala Trp Val Pro Phe Asp Pro Ala Ala Gln Ile Thr Lys Gln
   130 135 140
   Lys Trp Glu Ala Glu Pro Val Tyr Val Gln Arg Ala Lys Ala Tyr Leu
   145 150 155 160
   Glu Glu Glu Cys Pro Ala Thr Leu Arg Lys Tyr Leu Lys Tyr Ser Lys
   165 170 175
   Asn Ile Leu Asp Arg Gln Asp Pro Pro Ser Val Val Val Thr Ser His
   180 185 190
   Gln Ala Pro Gly Glu Lys Lys Lys Leu Lys Cys Leu Ala Tyr Asp Phe
   195 200 205
   Tyr Pro Gly Lys Ile Asp Val His Trp Thr Arg Ala Gly Gln Val Gln
   210 215 220
   Glu Pro Glu Leu Arg Gly Asp Val Leu His Asn Gly Asn Gly Thr Tyr
   225 230 235 240
   Gln Ser Trp Val Val Val Ala Val Pro Pro Gln Asp Thr Ala Pro Tyr
   245 250 255
   Ser Cys His Val Gln His Ser Ser Leu Ala Gln Pro Leu Val Val Pro
   260 265 270
   Trp Glu Ala Ser
   275
<210> 2
   <211> 4
   <212> PRT
   <213> Artificial sequence
<220>
   <223> Synthetic construct
<400> 2
<400> 2
<210> 3
   <211> 4
   <212> PRT
   <213> Artificial sequence
<220>
   <223> synthetic construct
<400> 3
   Asp Gly Val Asp
   1
<210> 4
   <211> 5
   <212> PRT
   <213> Artificial sequence
<220>
   <223> Synthetic construct
<400> 4
   Ile Glu Phe Thr Asp
   1 5
<210> 5
   <211> 4
   <212> PRT
   <213> Artificial sequence
<220>
   <223> Synthetic construct
<400> 5
   Asp Glu Val Asp
   1
<210> 6
   <211> 5
   <212> PRT
   <213> Artificial sequence
<220>
   <223> Synthetic construct
<400> 6
   Ile Glu Phe Thr Asp
   1               5